(19) Europäisches Patentamt
European Patent Office
Office européen des brevets

(11) EP 2 189 081 B1

(12) # FASCICULE DE BREVET EUROPEEN

(45) Date de publication et mention de la délivrance du brevet:
**10.05.2017 Bulletin 2017/19**

(21) Numéro de dépôt: **09175868.0**

(22) Date de dépôt: **12.11.2009**

(51) Int Cl.:
***A45D 40/00*** *(2006.01)*
***A61K 8/06*** *(2006.01)*
***A61K 8/31*** *(2006.01)*
***A61K 8/37*** *(2006.01)*
***A61K 8/81*** *(2006.01)*
***A61K 8/92*** *(2006.01)*
***A61Q 1/10*** *(2006.01)*
***A45D 40/26*** *(2006.01)*
***A61K 8/02*** *(2006.01)*

(54) **Composition cosmétique solide pour application sur les fibres kératiniques**

Feste kosmetische Zusammensetzung zum Auftragen auf Keratinfasern

Solid cosmetic composition for application to keratin fibres

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **24.11.2008 FR 0857976**
**10.12.2008 US 121291 P**

(43) Date de publication de la demande:
**26.05.2010 Bulletin 2010/21**

(73) Titulaire: **L'Oréal**
**75008 Paris (FR)**

(72) Inventeurs:
- **Arditty, Stéphane**
  **91160, Ballainvilliers (FR)**
- **Lahousse, Florence**
  **94320, Thiais (FR)**
- **Jager Lezer, Nathalie**
  **91370, Verrieres-Le-Buisson (FR)**

(74) Mandataire: **Nony**
**11 rue Saint-Georges**
**75009 Paris (FR)**

(56) Documents cités:
**EP-A- 0 662 312**
**EP-A- 1 410 787**
**EP-A- 1 745 771**
**EP-A- 1 894 602**
**EP-A- 1 913 835**
**WO-A-2008/046763**
**DE-A1- 10 233 288**
**DE-A1- 19 826 118**
**FR-A- 2 232 303**
**US-A- 5 389 363**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention se rapporte à une composition cosmétique de soin et/ou de maquillage sous forme de stick, destinée au soin et/ou au maquillage de fibres kératiniques et notamment de cils.

Par « fibres kératiniques », on entend les cils, les sourcils, les poils ou les cheveux.

Plus particulièrement, l'invention porte sur un mascara.

Par « mascara », on entend une composition destinée à être appliquée sur les fibres kératiniques, notamment sur les cils : il peut s'agir d'une composition de maquillage des cils, une base de maquillage des cils (aussi appelée base coat), une composition à appliquer sur un mascara (aussi appelée top-coat), ou bien encore une composition de traitement cosmétique des cils. Le mascara est plus particulièrement destiné aux cils d'êtres humains, mais également aux faux-cils.

D'une manière générale, on distingue deux types de mascara selon leur formulation : (i) des mascaras lavables ou mascaras « washable » qui peuvent être nettoyés avec de l'eau ou une eau savonneuse et qui sont présentés, généralement, sous la forme d'une émulsion de cires dans l'eau, telle que des crèmes ou des gels, et (ii) des mascaras résistants à l'eau ou mascaras « waterproof », nécessitant l'utilisation de formulation huileuse pour être éliminés et qui sont généralement formulés à l'état de dispersion de cire dans des solvants organiques.

La présente invention se rapport au type particulier de mascaras dits « washable ».

Les mascaras les plus classiques présentent habituellement une texture pâteuse et sont conditionnés dans un récipient comprenant un réservoir muni d'un essoreur et d'un applicateur, notamment sous forme d'une brosse ou d'un peigne, et qui s'appliquent par prélèvement du produit dans le réservoir à l'aide de l'applicateur, passage de l'applicateur au travers de l'essoreur afin d'évacuer le surplus de produit, puis mise en contact de l'applicateur imprégné de mascara sur les cils.

Il est également connu, par exemple des documents US 2,007,245 ou FR 2 833 163, des mascaras sous forme solide dits aussi « mascaras pains » qui sont des compositions comportant une forte proportion de cires, de pigments et de tensioactifs, délitables à l'eau, c'est à dire qu'ils nécessitent, préalablement à leur application sur les cils, la mise en contact avec une phase aqueuse de manière à solubiliser partiellement le pain de mascara. En particulier, l'application se fait *via* une brosse imprégnée d'eau qui est mise en contact avec le mascara puis le mélange prélevé et ensuite appliqué sur les cils avec la brosse de manière à déposer de la matière sur les cils.

Le document WO 2006/057439 décrit également des compositions de mascara solides à température ambiante qui sont ramollies à l'aide d'un dispositif augmentant la température de façon à pouvoir être appliquées sur les cils. Une fois la composition de mascara ramollie appliquée sur les cils, cette dernière est rapidement refroidie et ainsi se solidifie, permettant la formation d'un film ferme recouvrant les cils. Cette composition se présente sous la forme d'un mélange huileux dans lequel un composé solide hydrophobe forme la phase grasse. Le document FR2232303 décrit des compositions grasses pour la réalisation des produits de maquillage, comme un mascara automatique sous forme de pâte émulsifiée comprenant de la cire de carnauba. Cependant, la mise en oeuvre de ces compositions peut s'avérer longue et fastidieuse, nécessitant une bonne dextérité de l'utilisatrice. Autrement dit, plusieurs manipulations précèdent l'application en tant que telle sur les cils. De plus, de tels mascaras dits « mascaras pains » ou mascaras solides, peuvent nécessiter des dispositifs de mise en oeuvre particuliers comportant plusieurs éléments pouvant être coûteux.

Une autre voie de formulation pour une composition de revêtement des fibres kératiniques, notamment des cils, consiste en un stick qui permet en particulier une application pratique de la composition de revêtement sur les cils, par transfert direct de ladite composition sur les cils par simple application à sec, sans l'intermédiaire d'une brosse imprégnée d'eau ou d'un quelconque dispositif de chauffage.

Des sticks ont déjà été développés en cosmétique, notamment à des fins de maquillage et/ou de soin des matières kératiniques, dont certaines références sont citées ci-après. Parmi ces sticks, dédiés au maquillage et/ou au soin des matières kératiniques, certains se présentent sous la forme d'une émulsion eau-dans-huile solide comprenant une phase aqueuse émulsionnée avec un tensioactif dans une phase grasse, ladite phase grasse comprenant une huile, une cire et un copolymère séquencé. Le document EP 1 473 017 décrit un tel type d'émulsion inverse solide. Mais ces sticks présentent typiquement une dureté en texturométrie nettement supérieure à 5 000 g/m, à savoir trop dure pour être compatible avec une application plus spécifiquement sur les fibres kératiniques. Autrement dit, de tels sticks de l'art antérieur ne sont pas propices à l'application visée dans la présente demande.

La mise au point de compositions destinées au maquillage et/ou au soin des fibres kératiniques sous forme de stick applicable à sec, présente ainsi un certain nombre de difficultés. Ces dernières résident notamment dans l'obtention d'un stick de dureté contrôlée conditionnant la quantité de matière déposée sur les fibres kératiniques, notamment sur les cils, la qualité du dépôt, ainsi que la rapidité et la facilité du maquillage pour l'utilisatrice.

Le document EP 1 745 771 relatif à un procédé de revêtement de fibres kératiniques décrit des compostions sous forme de stick, applicables à sec et présentant une dureté allant de 500 à 18200 Pa, et exprimée selon le protocole décrit ci-après allant de 320 g/m à 11657 g/m. Cependant, les compositions illustrées dans ce document, non seulement visent des compositions anhydres de type « waterproof », mais de plus visent des compositions présentant des duretés

ne permettant pas l'obtention d'un stick propice à une application de type mascara répondant aux exigences de dureté contrôlée rappelées ci-dessus.

En effet, un stick trop dur ou trop mou, ne permettra pas un dépôt de qualité sur les cils notamment un dépôt suffisant et homogène, gainant de façon appropriée les cils et nuira à l'aspect pratique et rapide de l'application. Il est à noter qu'un stick trop dur ne permettra pas un dépôt suffisant de produit sur les cils, tandis qu'un stick trop mou risque d'engendrer un dépôt inhomogène, voire sous forme de paquets inesthétiques sur les cils. De plus, une pâte de produit trop molle nuira à l'élaboration d'un stick suffisamment résistant au choc et à la pression, notamment au moment de son application.

Il subsiste donc un besoin de disposer d'un stick suffisamment solide et rigide garantissant sa résistance au moment de son application, mais également suffisamment mou et délitable à sec afin de permettre un gainage approprié des cils comparable à celui obtenu à l'aide des mascaras les plus classiques actuellement sur le marché.

Les inventeurs ont maintenant découvert de façon surprenante qu'il était possible de disposer d'une composition répondant à ces exigences en élaborant une composition se présentant sous la forme d'une dispersion d'une phase grasse dans une phase aqueuse, caractérisée par sa dureté et par la présence de cire de Carnauba et/ou de cire d'abeille synthétique dans une teneur particulière. Les inventeurs ont ainsi découvert une composition de type « washable » présentant une dureté en cisaillement compatible avec la formation sur les cils d'un dépôt de qualité et d'épaisseur satisfaisante, gainant les cils de façon appropriée.

Selon un premier aspect, la présente invention vise une composition cosmétique de soin et/ou de maquillage des fibres kératiniques sous forme de stick, applicable à sec, (i) se présentant sous la forme d'une émulsion comportant une phase grasse dispersée dans une phase aqueuse, ladite phase grasse comprenant de la cire de Carnauba et/ou de la cire d'abeille synthétique, dans une teneur d'au moins 2 % en poids, de préférence d'au moins 3 % en poids, avantageusement d'au moins 4 % en poids, voire de 5 % à 30 % en poids, par rapport au poids total de ladite composition et (ii) présentant une dureté en cisaillement comprise entre 375 g/m et 5000 g/m, ladite dureté en cisaillement étant mesurée à 20 °C sur un stick cylindrique, à l'aide d'un fil rigide de diamètre 250 $\mu$m en tungstène en faisant avancer le fil relativement au stick à une vitesse de 100 mm/min.

La présence d'une teneur minimale de cires spécifiques telles que les cires de Carnauba et/ou d'abeille synthétique dans la composition selon l'invention, semblerait permettre une meilleure structuration de sa phase aqueuse rendant ainsi possible l'utilisation d'un extrait sec en quantité moindre dans ladite composition de type cire-dans-eau contrairement à l'utilisation d'autres cires qui nécessitent la présence d'un extrait sec supérieur à 45 % en poids par rapport au poids total de la composition portant préjudice à la qualité du dépôt sur les cils.

Selon un mode de réalisation particulier, la composition selon l'invention présente une teneur en extrait sec inférieure ou égale à 45 % en poids, en particulier inférieur à 45 % en poids, notamment supérieure ou égale à 25 % en poids, en particulier supérieure ou égale à 30 % en poids et encore plus particulièrement supérieure ou égale à 35 % en poids, par rapport au poids total de ladite composition.

Une telle composition est par ailleurs, avantageusement susceptible d'être obtenue, voire obtenue par un procédé comprenant au moins une étape comportant un apport de frigorie permettant un abaissement de la température de la composition à moins de 4°C, voire moins de 1°C, en moins de 30 minutes.

Selon un autre aspect, l'invention vise une composition cosmétique de soin et/ou de maquillage de fibres kératiniques, sous forme de stick, applicable à sec :

(i) se présentant sous la forme d'une émulsion comportant une phase grasse dispersée dans une phase aqueuse, ladite phase grasse comprenant de la cire de Carnauba et/ou de la cire d'abeille synthétique, dans une teneur d'au moins 2 % en poids, de préférence d'au moins 3 % en poids, avantageusement d'au moins 4 % en poids, voire de 5 % à 30 % en poids, par rapport au poids total de ladite composition,

(ii) présentant une dureté en cisaillement comprise entre 375 g/m et 5000 g/m, ladite dureté en cisaillement étant mesurée à 20 °C sur un stick cylindrique, à l'aide d'un fil rigide de diamètre 250 $\mu$m en tungstène en faisant avancer le fil relativement au stick à une vitesse de 100 mm/min, et

(iii) obtenue par un procédé comprenant au moins une étape comportant un apport de frigorie permettant un abaissement de la température de la composition à moins de 4°C, voire moins de 1°C, en moins de 30 minutes.

Selon encore un autre aspect, l'invention vise un procédé de revêtement de fibres kératiniques comprenant au moins les étapes consistant à :

- mettre lesdites fibres kératiniques au contact d'une partie au moins de la surface d'un stick comprenant une composition cosmétique selon l'invention; puis
- procéder à un déplacement relatif entre la surface dudit stick et lesdites fibres kératiniques, de manière à provoquer le délitage dudit stick et la formation d'un dépôt d'au moins une couche de ladite composition cosmétique sur lesdites fibres kératiniques.

Selon encore un autre aspect, l'invention vise un dispositif de conditionnement et d'application d'une composition cosmétique de soin et/ou de maquillage de fibres kératiniques, comprenant au moins :

a) un stick comprenant une composition cosmétique selon l'invention ;
b) un support dudit stick ; et
c) éventuellement au moins un élément d'application, ledit élément d'application pouvant être en outre muni d'organes de séparation et/ou de peignage desdites fibres kératiniques.

Selon encore un autre aspect, l'invention vise un stick destiné au maquillage et/ou au soin des fibres kératiniques, applicable à sec, susceptible d'être obtenu, voire obtenu par le procédé comprenant au moins les étapes suivantes consistant successivement à :

- chauffer une phase grasse et une phase aqueuse, ladite phase grasse comprenant de la cire de Carnauba et/ou de la cire d'abeille synthétique, dans une teneur d'au moins 2 % en poids par rapport au poids total de ladite composition à une température comprise entre 80 °C et 100 °C, plus particulièrement entre 85 °C et 100 °C, par exemple à une température de 95 °C,
- émulsionner lesdites phases à l'aide notamment d'un agitateur afin d'obtenir une composition de type émulsion à phase continue aqueuse,
- couler à chaud dans un dispositif de conditionnement ladite composition, puis
- refroidir ladite composition à une température comprise entre -30 °C et 10 °C, par exemple inférieure à -28 °C jusqu'à ce que la composition se présente sous la forme d'un stick, notamment pendant une durée comprise entre 15 et 60 minutes, par exemple 45 minutes.

Selon cet aspect, le stick présente avantageusement une dureté en cisaillement comprise entre 375 g/m et 5000 g/m, ladite dureté en cisaillement étant mesurée à 20 °C sur un stick à l'aide d'un fil rigide de diamètre 250 $\mu$m en tungstène en faisant avancer le fil relativement au stick à une vitesse de 100 mm/minute.

Selon un dernier aspect, l'invention concerne un stick destiné au maquillage et/ou au soin des fibres kératiniques, applicable à sec,

(i) se présentant sous la forme d'une émulsion comportant une phase grasse dispersée dans une phase aqueuse, ladite phase grasse comprenant de la cire de Carnauba et/ou de la cire d'abeille synthétique, dans une teneur d'au moins 2 % en poids, de préférence d'au moins 3 % en poids, avantageusement d'au moins 4 % en poids, voire de 5 % à 30 % en poids, par rapport au poids total de ladite composition,
(ii) présentant une dureté en cisaillement comprise entre 375 g/m et 5000 g/m, ladite dureté en cisaillement étant mesurée à 20 °C sur un stick à l'aide d'un fil rigide de diamètre 250 $\mu$m en tungstène en faisant avancer le fil relativement au stick à une vitesse de 100 mm/minute,
(iii) ledit stick étant obtenu par un procédé comprenant au moins les étapes suivantes consistant successivement à :

  - chauffer ladite émulsion à une température comprise entre 80 °C et 100 °C, plus particulièrement entre 85 °C et 100 °C, par exemple à une température de 95 °C,
  - émulsionner lesdites phases à l'aide notamment d'un agitateur afin d'obtenir une composition de type émulsion à phase continue aqueuse,
  - couler à chaud dans un dispositif de conditionnement ladite composition, puis
  - procéder à un apport de frigorie permettant un abaissement de la température de stick à moins de 4°C, voire moins de 1°C, en moins de 30 minutes.

**DEFINITIONS SELON L'INVENTION :**

Par « *stick* », on désigne un bâton, de forme prédéterminée qui, en l'absence de contrainte, à température ambiante et à pression atmosphérique, conserve sa forme prédéterminée. Ainsi conditionnée sous forme d'un stick, la composition est qualifiée d'auto-portée, de préférence pendant au moins 60 secondes. La section du stick peut être de toute forme, par exemple celle apparaissant sur la figure 1 annexée. La section peut être notamment en forme de « haricot », à savoir comporter une portion concave et une portion convexe vers l'intérieur, lesdites portions concave et convexe s'étendant selon un côté de plus grande dimension d'une section transversale. La section peut aussi être polygonale notamment carrée, rectangulaire ou encore ovale ou elliptique.

Par « *applicable à sec* », on entend que la composition est apte à former un dépôt, de préférence adhérent et gainant, sur les fibres kératiniques sans nécessiter de mise en contact préalable avec une phase aqueuse. La présente caractéristique « applicable à sec » délimite clairement la composition selon la présente invention des mascaras pains

qui sont délitables à l'eau et doivent au préalable être solubilisés partiellement pour être appliqués sur les fibres et former un dépôt adhérent et gainant.

Par « cylindre », on entend tous solides comprenant au moins deux faces opposées ou bases, éventuellement planes, le cas échéant parallèles, reliées entre elles par un ensemble de génératrices parallèles entre elles. Le terme « cylindre » comprend ainsi aussi bien les solides ayant au moins une base de contour fermé par une ligne directrice courbe que les solides de type prismes ayant au moins une base de contour fermé par une ligne directrice de type arête.

Au sens de la présente invention, la « *teneur en extrait sec* », désigne la teneur en matière non volatile.

La quantité d'extrait sec (abrégé ES) d'une composition selon l'invention est mesurée au moyen d'un dessiccateur à halogène commercial « HALOGEN MOISTURE ANALYZER HR83 » de chez METTLER TOLEDO. La mesure se fait sur la base de la perte de poids d'un échantillon séché par chauffage halogène et représente donc le pourcentage de matière résiduelle une fois que l'eau et les matières volatiles se sont évaporées.

Cette technique est parfaitement décrite dans la documentation de l'appareil fournie par METTLER TOLEDO.

Le protocole de mesure est le suivant :

On étale environ 2 g de la composition, ci-après l'échantillon, sur une coupelle métallique que l'on introduit dans le dessiccateur à halogène mentionné ci-dessus. L'échantillon est alors soumis à une température de 120 °C pendant une heure. La Masse Humide de l'échantillon, correspondant à sa masse initiale, et la Masse Sèche de l'échantillon, correspondant à sa masse après chauffage halogène, sont mesurées au moyen d'une balance de précision.

L'erreur expérimentale liée à la mesure est de l'ordre de plus ou moins 2 %. La teneur en Extrait Sec est calculée de la manière suivante :

**Teneur en Extrait Sec** (exprimé en % en poids) = 100 x (Masse Sèche / Masse Humide).

On entend par « *polymère* » des composés comportant au moins deux motifs, de préférence au moins 3 motifs et plus spécialement au moins 10 motifs de répétition.

La figure 1 montre une vue de profil en perspective d'un mode de réalisation préférentiel d'un dispositif 1 selon l'invention, le bouton poussoir étant en position médiane. Le dispositif de conditionnement préféré représenté à la figure 1 n'est présenté qu'à titre indicatif et nullement limitatif de l'invention.
La figure 2 constitue une vue en cours d'utilisation d'un dispositif 1 selon l'invention.

L'invention va maintenant être décrite plus en détails.

## DURETE

Une composition cosmétique de soin et/ou de maquillage de fibres kératiniques sous forme de stick, applicable à sec, selon l'invention présente une dureté en cisaillement comprise entre 375 g/m et 5 000 g/m mesurée selon la méthode définie ci-dessous.

Avec une telle dureté, la composition est assez « molle » pour permettre une application directe et aisée sur les cils, notamment un dépôt de matière par simple mise en contact avec les cils, sans exercer une pression exagérée sur la frange de cils.

Pour déterminer la « *dureté en cisaillement* » d'un stick conforme à l'invention, on peut utiliser la méthode dite « du fil à couper le beurre », qui consiste à couper transversalement un stick cylindrique, par exemple de diamètre 8 mm à l'aide d'un fil rigide de diamètre 250 μm en tungstène en faisant avancer le fil relativement au stick à une vitesse de 100 mm/min. La dureté correspond à la force maximale de cisaillement exercée par le fil sur le stick à 20 °C, cette force étant mesurée au moyen d'un dynamomètre DFGS2 commercialisé par la société INDELCO-CHATILLON. La mesure est reproduite 6 fois. La moyenne des 6 valeurs lues au moyen du dynamomètre mentionné ci-dessus, est exprimée en grammes. Ces valeurs sont ainsi comprises entre 3 g et 40 g, avantageusement entre 4 g et 20 g et de préférence entre 6 g et 12 g. Cette opération est effectuée sur la plus grande longueur de la section du stick lorsque le stick se présente sous forme de cylindre de section transversale allongée. Puis, on normalise cette valeur en grammes par cette plus grande longueur, ou le cas échéant par le diamètre du stick lorsque le stick se présente sous forme de cylindre de section transversale circulaire, soit 8 mm par exemple, afin d'obtenir une valeur en grammes / mètre.

Autrement dit, la valeur de la dureté selon la présente invention correspond à la force maximale de cisaillement exercée par le fil mesurée sur le stick à 20 °C divisée par la plus grande longueur de la section dudit stick, mise en contact avec ledit fil de 250 μm de diamètre.

Selon un mode de réalisation particulier, la composition selon l'invention est caractérisée en ce que la dureté en cisaillement est comprise entre 500 g/m et 2500 g/m, en particulier entre 750 g/m et 1500 g/m.

La composition selon l'invention se présente sous la forme d'une émulsion, notamment d'une émulsion comportant une phase grasse dispersée dans une phase aqueuse, en particulier une émulsion cire-dans-eau.

La composition selon l'invention comprend ainsi une phase aqueuse continue.

## PHASE AQUEUSE

La composition selon l'invention comprend une phase aqueuse qui peut être constituée essentiellement d'eau ou qui peut comprendre un mélange d'eau et de solvant miscible à l'eau ou hydrosoluble (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$, le glycérol et leur mélanges.

Selon un mode de réalisation, la composition comprend une phase aqueuse en une teneur supérieure à 40 %, de préférence supérieure à 45 % en poids, et mieux supérieure à 50 % en poids, et/ou une teneur inférieure à 80 % en poids, de préférence inférieure à 75 % en poids, de préférence allant de 40 % à 80 % en poids, et notamment allant de 50 % à 75 % en poids, par rapport au poids total de la composition.

La composition selon l'invention peut, en outre, contenir au moins un agent émulsionnant.

## Agents émulsionnants

Selon l'invention, on utilise généralement un agent émulsionnant choisi de manière appropriée pour l'obtention d'une émulsion cire-dans-eau ou huile-dans-eau. En particulier, on peut utiliser un agent émulsionnant possédant à 25 °C une balance HLB (hydrophile-lipophile balance) au sens de GRIFFIN, supérieure ou égale à 8.

La valeur HLB selon GRIFFIN est définie dans J. Soc. Cosm. Chem. 1954 (volume 5), pages 249-256.

Ces agents émulsionnants peuvent être choisis parmi des tensioactifs non ioniques, anioniques, cationiques, amphotériques ou encore des tensioactifs polymériques. On peut se reporter au document « Encyclopedia of Chemical Technology, KIRK-OTHMER », volume 22, p. 333-432, 3ème édition, 1979, WILEY, pour la définition des propriétés et des fonctions (émulsionnant) des tensioactifs, en particulier p. 347-377 de cette référence, pour les tensioactifs anioniques, amphotériques et non ioniques.

Les tensioactifs utilisés préférentiellement dans la composition selon l'invention sont choisis parmi :

a) les tensioactifs non ioniques de HLB supérieur ou égal à 8 à 25 °C, utilisés seuls ou en mélange; on peut citer notamment :

- les esters et éthers d'oses tels que le mélange de cétylstéaryl glucoside et d'alcools cétylique et stéarylique comme le Montanov 68 de Seppic;
- les éthers oxyéthylénés et/ou oxypropylénés (pouvant comporter de 1 à 150 groupes oxyéthylénés et/ou oxypropylénés) de glycérol ;
- les éthers oxyéthylénés et/ou oxypropylénés (pouvant comporter de 1 à 150 groupes oxyéthylénés et/ou oxypropylénés) d'alcools gras (notamment d'alcool en $C_8$-$C_{24}$, et de préférence en $C_{12}$-$C_{18}$) tels que l'éther oxyéthyléné de l'alcool cétéarylique à 30 groupes oxyéthylénés (nom CTFA « Ceteareth-30 »), l'éther oxyéthyléné de l'alcool stéarylique à 20 groupes oxyéthylénés (nom CTFA « Steareth-20 »), l'éther oxyéthyléné du mélange d'alcools gras en $C_{12}$-$C_{15}$ comportant 7 groupes oxyéthylénés (nom CTFA « $C_{12-15}$ Pareth-7 ») notamment commercialisé sous la dénomination NEODOL 25-7® par

SHELL CHEMICALS

- les esters d'acide gras (notamment d'acide en $C_8$-$C_{24}$, et de préférence en $C_{16}$-$C_{22}$) et de polyéthylène glycol (pouvant comprendre de 1 à 150 motifs d'éthylèneglycol) tels que le stéarate de PEG-50 et le monostéarate de PEG-40, notamment commercialisé sous le nom MYRJ 52P® par la société ICI UNIQUEMA,
- les esters d'acide gras (notamment d'acide en $C_8$-$C_{24}$, et de préférence en $C_{16}$-$C_{22}$) et des éthers de glycérol oxyéthylénés et/ou oxypropylénés (pouvant comporter de 1 à 150 groupes oxyéthylénés et/ou oxypropylénés), comme le monostéarate de PEG-200 glycéryle notamment vendu sous la dénomination Simulsol 220 TM® par la société SEPPIC ; le stéarate de glycéryle polyéthoxylé à 30 groupes d'oxyde d'éthylène comme le produit TAGAT S® vendu par la société Evonik GOLDSCHMIDT, l'oléate de glycéryle polyéthoxylé à 30 groupes d'oxyde d'éthylène

comme le produit TAGAT O® vendu par la société Evonik GOLDSCHMIDT, le cocoate de glycéryle polyéthoxylé à 30 groupes d'oxyde d'éthylène comme le produit VARIONIC LI 13® vendu par la société SHEREX, l'isostéarate de glycéryle polyéthoxylé à 30 groupes d'oxyde d'éthylène comme le produit TAGAT L® vendu par la société Evonik GOLDSCHMIDT et le laurate de glycéryle polyéthoxylé à 30 groupes d'oxyde d'éthylène comme le produit TAGAT I® de la société Evonik GOLDSCHMIDT,

- les esters d'acide gras (notamment d'acide en $C_8$-$C_{24}$, et de préférence en $C_{16}$-$C_{22}$) et des éthers de sorbitol oxyéthylénés et/ou oxypropylénés (pouvant comporter de 1 à 150 groupes oxyéthylénés et/ou oxypropylénés), comme le polysorbate 20 notamment vendu sous la dénomination Tween 20® par la société CRODA, le polysorbate 60 notamment vendu sous la dénomination Tween 60® par la société CRODA,
- la diméthicone copolyol, telle que celle vendue sous la dénomination Q2-5220® par la société DOW CORNING,
- la diméthicone copolyol benzoate (FINSOLV SLB 101® et 201® de la société FINTEX),
- les copolymères d'oxyde de propylène et d'oxyde d'éthylène, également appelés polycondensats OE/OP,
- et leurs mélanges.

Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante :

$$H\text{-}(O\text{-}CH_2\text{-}CH_2)_a\text{-}(O\text{-}CH(CH_3)\text{-}CH_2)_b\text{-}(O\text{-}CH_2\text{-}CH_2)_a\text{-}OH,$$

formule dans laquelle a va de 2 à 120, et b va de 1 à 100.

Le polycondensat OE/OP a de préférence un poids moléculaire moyen en poids allant de 1000 à 15000, et mieux allant de 2000 à 13000. Avantageusement, ledit polycondensat OE/OP a une température de trouble, à 10 g/l en eau distillée, supérieure ou égale à 20 °C, de préférence supérieure ou égale à 60 °C. La température de trouble est mesurée selon la norme ISO 1065. Comme polycondensat OE/OP utilisable selon l'invention, on peut citer les polycondensats tribloc polyéthylène glycol / polypropylène glycol / polyéthylène glycol vendus sous les dénominations SYNPERONIC® comme les SYNPERONIC PE/ L44® et SYNPERONIC PE/F127® par la société ICI.

b) les tensioactifs non ioniques de HLB inférieur à 8 à 25 °C, éventuellement associés à un ou plusieurs agents tensioactif non ioniques de HLB supérieur à 8 à 25 °C tels que cités ci-dessus ; on peut citer notamment :

- les esters et éthers d'oses tels que les stéarate de sucrose, cocoate de sucrose, stéarate de sorbitan et leurs mélanges comme l'Arlatone 2121® commercialisé par la société ICI ;
- les éthers oxyéthylénés et/ou oxypropylénés (pouvant comporter de 1 à 150 groupes oxyéthylénés et/ou oxypropylénés) d'alcools gras (notamment d'alcool en $C_8$-$C_{24}$, et de préférence en $C_{12}$-$C_{18}$) tels que l'éther oxyéthyléné de l'alcool stéarylique à 2 groupes oxyéthylénés (nom CTFA « Steareth-2 ») ;
- les esters d'acides gras (notamment d'acide en $C_8$-$C_{24,}$ et de préférence en $C_{16}$-$C_{22}$) et de polyol, notamment de glycérol ou de sorbitol, tels que stéarate de glycéryle, tel que le produit vendu sous la dénomination TEGIN M® par la société Evonik GOLDSCHMIDT, laurate de glycéryle tel que le produit vendu sous la dénomination IMWITOR 312® par la société HULS, stéarate de polyglycéryl-2, tristéarate de sorbitan, ricinoléate de glycéryle ;
- les lécithines, telles que les lécithines de soja (comme Emulmetik 100 J de Cargill, ou Biophilic H de Lucas Meyer) ;
- le mélange de cyclométhicone/diméthicone copolyol vendu sous la dénomination Q2-3225C® par la société DOW CORNING.

c) les tensioactifs anioniques tels que :

- les sels d'acides gras en $C_{16}$-$C_{30}$ notamment ceux dérivant des amines, comme le stéarate de triéthanolamine et/ou le stéarate d'amino-2-méthyl-2-propane di-ol-1,3. Le stéarate de triéthanolamine est généralement obtenu par simple mélange de l'acide stéarique et de la triéthanolamine ;
- les sels d'acides gras polyoxyéthylénés notamment ceux dérivant des amines ou les sels alcalins, et leurs mélanges ;
- les esters phosphoriques et leurs sels tels que le « DEA oleth-10 phosphate » (Crodafos N 10N de la société CRODA) ou le phosphate de monocétyle monopotassique ou potassium cétylphosphate (Amphisol K de Crivaudan) ;
- les sulfosuccinates tels que le « Disodium PEG-5 citrate lauryl sulfosuccinate » et le « Disodium ricinoleamido MEA sulfosuccinate » ;

- les alkyléthersulfates tels que le lauryl éther sulfate de sodium ;
- les iséthionates ;
- les acylglutamates tels que le « Disodium hydrogenated tallow glutamate » (AMISOFT HS-21 R® commercialisé par la société AJINOMOTO) et le sodium stearoyl glutamate (AMISOFT HS-11 PF® commercialisé par la société AJINOMOTO) et leurs mélanges ;
- les dérivés de soja comme le potassium soyate ;
- les citrates, comme le Glyceryl stearate citrate (Axol C 62 Pellets de Degussa) ;
- les dérivés de proline, comme le Sodium palmitoyl proline (Sepicalm VG de Seppic), ou le Mélange de Sodium palmitoyle sarcosinate, Magnesium palmitoyle glutamate, acide palmitique et Palmitoyle proline (Sepifeel One de Seppic) ;
- les lactylates, comme le Sodium stearoyl lactylate (Akoline SL de Karlshamns AB) ;
- les sarcosinates, comme le sodium palmitoyle sarcosinate (Nikkol sarcosinate PN) ou le mélange de Stéaroyle sarcosine et Myristoyle sarcosine 75/25 (Crodasin SM de Croda) ;
- les sulfonates, comme le Sodium C14-17 alkyl sec sulfonate (Hostapur SAS 60 de Clariant) ;
- les glycinates, comme le sodium cocoyle glycinate (Amilite GCS-12 d'Ajinomoto).

Les compositions conformes à l'invention peuvent également contenir un ou plusieurs tensioactifs amphotériques comme les N-acyl-aminoacides tels que les N-alkyl-aminoacétates et le cocoamphodiacetate disodique et les oxydes d'amines tels que l'oxyde de stéaramine ou encore des tensioactifs siliconés comme les diméthicone copolyols phosphates tels que celui vendu sous la dénomination PECOSIL PS 100® par la société PHOENIX CHEMICAL.

L'agent émulsionnant utilisable peut également être un tensioactif polymérique, notamment un polymère thermogélifiant.

Selon un mode de réalisation particulier de l'invention, l'agent émulsionnant est choisi parmi (i) les sels d'acides gras en $C_{16}$-$C_{30}$ notamment ceux dérivant des amines, comme le stéarate de triéthanolamine; (ii) les éthers oxyéthylénés et/ou oxypropylénés (pouvant comporter de 1 à 150 groupes oxyéthylénés et/ou oxypropylénés) d'alcools gras (notamment d'alcool en $C_8$-$C_{24}$, et de préférence en $C_{12}$-$C_{18}$) tels que l'éther oxyéthyléné de l'alcool stéarylique à 2 groupes oxyéthylénés (nom CTFA « Steareth-2 ») ; (iii) les esters d'acides gras (notamment d'acide en $C_8$-$C_{24}$, et de préférence en $C_{16}$-$C_{22}$) et de polyol, notamment de glycérol ou de sorbitol, tels que le stéarate de glycéryle tel que le produit vendu sous la dénomination TEGIN M® par la société Evonik GOLDSCHMIDT; (iv) les esters phosphoriques et leurs sels tels que le potassium cétylphosphate (Amphisol K de Givaudan) et/ou (v) les esters d'acide gras (notamment d'acide en $C_8$-$C_{24}$, et de préférence en $C_{16}$-$C_{22}$) et des éthers de glycérol oxyéthylénés et/ou oxypropylénés (pouvant comporter de 1 à 150 groupes oxyéthylénés et/ou oxypropylénés), comme le stéarate de glycéryle polyéthoxylé à 30 groupes d'oxyde d'éthylène comme le produit TAGAT® S vendu par la société Evonik GOLDSCMIDT et (vi) leurs mélanges.

Convient encore plus particulièrement à titre d'agent émulsionnant conforme à l'invention, le steareth-2, le stéarate de glycéryl, le stéarate de triéthanolamine, le stéarate de glycéryle polyéthoxylé à 30 groupes d'oxyde d'éthylène, le potassium cétylphosphate ou leurs mélanges.

Selon un mode de réalisation, la composition selon l'invention comprend au moins un tensioactif anionique et éventuellement au moins un tensioactif non ionique, en particulier un tensioactif non ionique de HLB supérieur ou égal à 8 à 25 °C, lesdits tensioactifs pouvant être avantageusement choisis parmi les tensioactifs mentionnés ci-dessus.

Toujours selon ce mode de réalisation, la composition selon l'invention comprend au moins un tensioactif anionique choisi parmi les sels d'acides gras en $C_{16}$-$C_{30}$ notamment ceux dérivant des amines, comme le stéarate de triéthanolamine ; les esters phosphoriques et leurs sels tels que le potassium cétylphosphate (Amphisol K de Givaudan) et leurs mélanges et éventuellement au moins un tensioactif non ionique choisi parmi les éthers oxyéthylénés et/ou oxypropylénés (pouvant comporter de 1 à 150 groupes oxyéthylénés et/ou oxypropylénés) d'alcools gras (notamment d'alcool en $C_8$-$C_{24}$, et de préférence en $C_{12}$-$C_{18}$) tels que l'éther oxyéthyléné de l'alcool stéarylique à 2 groupes oxyéthylénés (nom CTFA « Steareth-2 ») ; les esters d'acides gras (notamment d'acide en $C_8$-$C_{24}$, et de préférence en $C_{16}$-$C_{22}$) et de polyol, notamment de glycérol ou de sorbitol, tels que le stéarate de glycéryle tel que le produit vendu sous la dénomination TEGIN M® par la société Evonik GOLDSCHMIDT; les esters d'acide gras (notamment d'acide en $C_8$-$C_{24}$, et de préférence en $C_{16}$-$C_{22}$) et des éthers de glycérol oxyéthylénés et/ou oxypropylénés (pouvant comporter de 1 à 150 groupes oxyéthylénés et/ou oxypropylénés), comme le stéarate de glycéryle polyéthoxylé à 30 groupes d'oxyde d'éthylène comme le produit TAGAT® S vendu par la société Evonik GOLDSCMIDT et leurs mélanges.Selon un mode de réalisation, la composition selon l'invention comprend au moins un agent émulsionnant choisi parmi le potassium cétylphosphate, le stéareth-2 et 20, alcool cétylique et leur mélange. Avantageusement, il s'agit d'un mélange de potassium cétylphosphate et de stéareth-2.

La composition selon l'invention peut contenir de 0,01 à 30 % en poids d'agent émulsionnant, par rapport au poids total de ladite composition, mieux de 1 à 15 % en poids et mieux encore de 2 à 10 % en poids.

La composition selon l'invention peut comprendre, en outre, au moins un gélifiant hydrophile.

**Gélifiants hydrophiles**

Les gélifiants hydrophiles utilisables dans les compositions selon l'invention peuvent être choisi parmi :

- les homo- ou copolymères d'acides acrylique ou méthacrylique, leurs sels et leurs esters, en particulier les produits vendus sous les dénominations VERSICOL F® ou VERSICOL K® par la société ALLIED COLLOID, UTRAHOLD 8® par la société CIBA-GEIGY, les acides polyacryliques de type SYNTHALEN K®,
- les copolymères d'acide acrylique et d'acrylamide vendus sous la forme de leur sel de sodium sous les dénominations RETEN® par la société HERCULES, le polyméthacrylate de sodium vendu sous la dénomination DARVAN N°7® par la société VANDERBILT, les sels de sodium d'acides polyhydroxycarboxyliques vendus sous la dénomination HYDAGEN F® par la société HENKEL,
- les copolymères acide polyacryliques/acrylates d'alkyle de type PEMULEN®,
- l'AMPS (Acide polyacrylamidométhyl propane sulfonique neutralisé partiellement à l'ammoniaque et hautement réticulé) commercialisé par la société CLARIANT,
- les copolymères AMPS/acrylamide de type SEPIGEL® ou SIMULGEL® commercialisés par la société SEPPIC, et
- les copolymères AMPS/méthacrylates d'alkyle polyoxyéthylénés (réticulés ou non), et leurs mélanges.

Les polymères filmogènes hydrosolubles cités plus bas peuvent également jouer le rôle de gélifiant hydrophile.

Le ou les gélifiant(s) hydrophile(s) peuvent être présent(s) dans la composition selon l'invention en une teneur en matières sèches allant de 0,01 % à 30 % en poids, de préférence de 0,5 % à 20 % en poids, mieux de 1 % à 15 % en poids par rapport au poids total de ladite composition.

**PHASE GRASSE**

La composition selon l'invention comprend une phase grasse, comprenant au moins de la cire de Carnauba, de la cire d'abeille synthétique ou leur mélange, susceptible(s) de servir d'agent(s) texturant(s) de la composition.

Par « agent texturant », on entend un composé capable d'apporter de la texture à la composition, notamment en formant un réseau agrégé de particules.

Un tel agent texturant reste non miscible à la phase aqueuse, et peut se présenter sous forme de dispersion dans ladite phase aqueuse. Un tel agent texturant, lorsqu'il est présent en quantité suffisante, conduit ainsi à des textures semi-solide ou solide.

La cire de Carnauba et/ou la cire d'abeille synthétique sont présentes dans la composition selon l'invention en une teneur d'au moins 2 % en poids, avantageusement d'au moins 3 % en poids, mieux encore d'au moins 4 % en poids, notamment de 2 à 30 %, par exemple de 2 à 25 %, ou encore de 2 à 10 % notamment lorsque la composition comprend des cires additionnelles, ou bien de 15 à 25 %, notamment lorsque la composition ne comprend pas de cire additionnelles, voire de 5 à 30 % en poids par rapport au poids total de ladite composition.

Par cire de Carnauba et/ou cire d'abeille synthétique, on entend dans le cadre de la présente invention, la cire de Carnauba, la cire d'abeille synthétique ou leur mélange.

Sans que l'invention soit liée à une quelconque théorie, les inventeurs ont émis l'hypothèse que la caractéristique liée à la présence dans l'émulsion cire-dans-eau de la cire de Carnauba et/ou la cire d'abeille synthétique dans une teneur d'au moins 2% en poids par rapport au poids total de l'émulsion, assortie de l'apport de frigorie conforme à l'invention, permet à un émulsion cire-dans-eau d'atteindre la dureté requise pour l'application spécifiquement recherchée dans le cadre de la présente invention, par un agencement particulier des particules de cire. Les inventeurs ont plus particulièrement émis l'hypothèse que ce choix de cires dans cette teneur minimale requise, permet d'obtenir une morphologie des cristaux particulière, notamment au moins en partie sous forme d'aiguilles et ainsi l'obtention d'un réseau d'un rigidité adéquate, permettant un piégeage des molécules d'eau particulièrement adapté aux exigences de dureté et propice à l'obtention de bonnes propriétés de maquillage.

Autrement dit, l'organisation des cristaux de cire participe pleinement à la structuration du stick et distingue clairement le stick obtenu dans le cadre de la présente invention, d'une émulsion traditionnelle de mascara, déformable et applicable par des applicateurs classiques, par exemple des brosses.

**Cire de Carnauba**

La cire de Carnauba contient généralement des esters d'acide gras, par exemple à hauteur de 80 à 85 % en poids, des alcools gras, par exemple à hauteur de 10 à 15 % en poids, des acides, par exemple à hauteur de 3 à 6 % en poids, et des chaînes hydrocarbonées, par exemple à hauteur de 1 à 3 % en poids, par rapport au poids total de la cire. Elle peut en particulier contenir un fort taux d'ester de diol, notamment environ 20 % en poids par rapport au poids total de la cire, d'acide gras hydroxylé, notamment environ 6 % en poids et d'acide cinnamique, notamment environ 10

% en poids, par rapport au poids total de la cire.

Les esters d'acides gras à hauteur de 80 à 85 % en poids peuvent comprendre de 36 à 40 % en poids de monoesters, de 26 à 34 % de diesters d'acide cinnamique et de 10 à 14 % en poids d'hydroxyesters.

Son point de fusion est d'environ 78 à 85 °C.

La cire de Carnauba est notamment vendue par la société Strahl & Pitch sous la dénomination commerciale Carnauba Wax SP 63®, ou encore par la société Baerlocher sous la dénomination commerciale Cerauba® T1.

**Cire d'abeille synthétique**

La cire d'abeille synthétique, de nom INCI Synthetic Beeswax, contient généralement des esters d'alcools et d'acides gras en $C_{16}$ à $C_{36}$. Son point de fusion est d'environ 50 à 60 °C.

La cire d'abeille synthétique est notamment vendue par la société Evonik Goldschmidt sous la dénomination commerciale Cyclochem® 326 A.

Selon un mode de réalisation particulier, la composition peut être exempte de cires additionnelles. La teneur en cire de Carnauba et/ou cire d'abeille synthétique peut alors être comprise entre 15 % et 30 % en poids, par exemple entre 15 % et 25 % en poids, par rapport au poids total de la composition.

La composition selon l'invention peut comprendre, en outre, au moins un agent dispersé additionnel, texturant ou non, qui peut être choisi parmi (i) des cires additionnelles, autres que la cire de Carnauba et/ou la cire d'abeille synthétique, (ii) des polymères semi-cristallins, (iii) des gélifiants lipophiles, et (iv) leurs mélanges.

Le ou les agent(s) dispersé(s) additionnel(s) peuvent représenter de 1 à 40 % en poids, par rapport au poids total de la composition selon l'invention, de préférence de 2,5 à 30 % et de façon encore plus préférée de 5 à 25 % en poids. La quantité en agent dispersé additionnel peut être ajustée, par l'homme du métier, en fonction des propriétés de structuration desdits agents.

La composition selon l'invention peut ainsi comprendre, en outre, au moins cire additionnelle.

Cire(s) additionnelles

Les cires additionnelles considérées dans le cadre de la présente invention sont d'une manière générale des composés lipophiles, solides, déformables ou non déformables, à température ambiante (25 °C), à changement d'état solide/liquide réversible, ayant un point de fusion supérieur ou égal à 30 °C pouvant aller jusqu'à 200 °C et notamment jusqu'à 120 °C.

Lorsqu'une phase grasse liquide est présente, en portant une ou des cires conforme(s) à l'invention à l'état liquide (fusion), il est possible de la ou les rendre miscibles à une ou des huiles et de former un mélange cire(s) + huile(s), homogène macroscopiquement, mais en ramenant la température dudit mélange à la température ambiante, on obtient une recristallisation de la ou des cire(s) dans la ou les huile(s) du mélange.

En particulier, les cires additionnelles convenant à l'invention peuvent présenter un point de fusion supérieur ou égal à 45 °C, et en particulier supérieur ou égal à 55 °C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de cire, disposé dans un creuset, est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin est soumis à une deuxième montée en température allant de 20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires additionnelles susceptibles d'être utilisées dans les compositions selon l'invention sont choisies parmi les cires, solides à température ambiante, d'origine animale, végétale, minérale ou de synthèse et leurs mélanges.

On peut notamment utiliser les cires hydrocarbonées comme la cire d'abeilles naturelle (ou cire d'abeille blanchie), la cire de lanoline, et les cires d'insectes de Chine; la cire de riz, la cire de Candellila, la cire d'Ouricurry, la cire d'Alfa, la cire de fibres de liège, la cire de canne à sucre, la cire du Japon et la cire de sumac; la cire de montan, les cires micro cristallines, les paraffines et l'ozokérite; les cires de polyéthylène, les cires obtenues par la synthèse de Fisher-Tropsch et les copolymères cireux ainsi que leurs esters.

On peut aussi citer les cires obtenues par hydrogénation catalytique d'huiles animales ou végétales ayant des chaînes grasses, linéaires ou ramifiées, en $C_8$-$C_{32}$.

Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) notamment vendu sous la dénomination « HEST 2T-4S » par la société HETERENE, le tétrabéhénate de di-(triméthylol-1,1,1 propane) notamment vendue sous la dénomination HEST 2T-4B par la société HETERENE.

On peut encore citer les cires de silicone comme les alkyl ou alkoxy-diméthicone ayant de 16 à 45 atomes de carbone, les cires fluorées.

On peut également utiliser la cire obtenue par hydrogénation d'huile d'olive estérifiée avec l'alcool stéarylique notamment vendue sous la dénomination « PHYTOWAX Olive 18 L 57 » ou bien encore les cires obtenues par hydrogénation d'huile de ricin estérifiée avec l'alcool cétylique notamment vendus sous la dénomination « PHYTOWAX ricin 16L64 et 22L73 », par la société SOPHIM. De telles cires sont décrites dans la demande FR-A-2 792 190.

Selon un mode de réalisation particulier, les compositions conformes à l'invention peuvent comprendre au moins une cire dite cire collante c'est-à-dire possédant un collant supérieur ou égal à 0,7 N.s et une dureté inférieure ou égale à 3,5 MPa.

L'utilisation d'une cire collante peut notamment permettre l'obtention d'une composition cosmétique qui s'applique facilement sur les cils, ayant une bonne accroche sur les cils et qui conduit à la formation d'un maquillage lisse, homogène et épaississant.

La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s à 10 N.s, et en particulier allant de 2 N.s à 5 N.s.

Le collant de la cire est déterminé par la mesure de l'évolution de la force (force de compression ou force d'étirement) en fonction du temps, à 20 °C à l'aide du texturomètre vendu sous la dénomination « TA-TX2i® » par la société RHEO, équipé d'un mobile en polymère acrylique en forme de cône formant un angle de 45°.

Le protocole de mesure est le suivant :

La cire est fondue à une température égale au point de fusion de la cire +10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.

Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.

La dureté de la cire collante est déterminée par la mesure de la force en compression mesurée à 20 °C à l'aide du texturomètre vendu sous la dénomination TA-XT2 par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure de la dureté ou du collant.
Le mobile du texturomètre est déplacé à la vitesse de 0,1 mm/s, puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,5 mm/s.

La valeur de la dureté est la force de compression maximale mesurée divisée par la surface du cylindre du texturomètre en contact avec la cire.

Comme cire collante, on peut utiliser un (hydroxystéaryloxy)stéarate d'alkyle en $C_{20}$-$C_{40}$ (le groupe alkyle comprenant de 20 à 40 atomes de carbone), seul ou en mélange, en particulier un 12-(12'-hydroxystéaryloxy)stéarate

d'alkyle en $C_{20}$-$C_{40}$, de formule (II) :

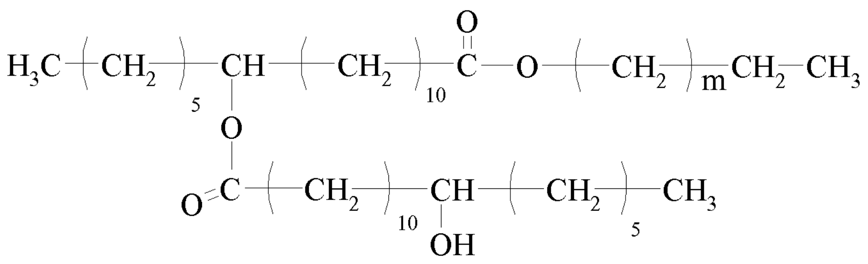

(II)

dans laquelle m est un entier allant de 18 à 38, ou un mélange de composés de formule (II).

Une telle cire est notamment vendue sous les dénominations « Kester Wax K 82 P® » et « Kester Wax K 80 P® » par la société KOSTER KEUNEN.

On peut également utiliser la cire microcristalline commercialisée sous la référence SP18 par la société STRAHL and PITSCH qui présente une dureté d'environ 0,46 MPa et une valeur de collant d'environ 1 N.s.

La ou les cires peu(ven)t être présente(s) sous forme d'une microdispersion aqueuse de cire. On entend par microdispersion aqueuse de cire, une dispersion aqueuse de particules de cire, dans laquelle la taille desdites particules de cire est inférieure ou égale à environ 1 $\mu$m.

Les microdispersions de cire sont des dispersions stables de particules colloïdales de cire, et sont notamment décrites dans « Microemulsions Theory and Practice », L.M. Prince Ed., Academic Press (1977) pages 21-32.

En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.

Les microdispersions de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 $\mu$m (notamment allant de 0,02 $\mu$m à 0,99 $\mu$m), de préférence inférieures à 0,5 $\mu$m (notamment allant de 0,06 $\mu$m à 0,5 $\mu$m).

Ces particules sont constituées essentiellement d'une cire ou d'un mélange de cires. Elles peuvent toutefois comprendre en proportion minoritaire des additifs gras, huileux et/ou pâteux, un tensioactif et/ou un additif/actif liposoluble usuel.

Selon un mode de réalisation, la composition comprend en outre au moins une cire additionnelle, autre que la cire d'abeille synthétique et de Carnauba, choisie parmi les cires, solides à température ambiante, éventuellement sous forme de micro dispersion aqueuse de cire, d'origine animale, végétale, minérale ou de synthèse et leurs mélanges. Toujours selon ce mode de réalisation, au moins une cire additionnelle peut présenter un point de fusion supérieur ou égal à 45 °C, en particulier supérieur ou égal à 55 °C et/ou posséder un collant supérieur ou égal à 0,7 N.s et une dureté inférieure ou égale à 3,5 MPa.

Selon un mode de réalisation particulier, la composition selon l'invention peut comprendre à titre de cire additionnelle au moins de l'ozokérite. En effet, les inventeurs ont constaté que la présence d'ozokérite est avantageuse car elle engendre une propriété de non adhérence aux parois de l'article le contenant. Autrement dit, sa présence permet d'éviter un rétrain du stick au refroidissement, évitant une trop forte adhérence de celui-ci aux parois de l'article de conditionnement et, de plus, est propice à un état de surface du stick, lisse et brillant.

Selon ce mode de réalisation, la composition selon l'invention peut comprendre de 5 % à 25 % en poids d'ozokérite, en particulier de 10 % à 20 % en poids, par rapport au poids total de la composition.

La composition selon l'invention peut présenter une teneur totale en cire(s), c'est-à-dire en cire de Carnauba et/ou d'abeille synthétique et en cire(s) additionnelle(s), comprise entre 2 et 35 % en poids, de préférence entre 5 et 30 % en poids, par rapport au poids total de ladite composition.

La composition selon l'invention peut comprendre, en outre, au moins un polymère semi-cristallin.

## <u>Polymères semi-cristallins</u>

Par « polymère semi-cristallin », on entend des polymères comportant une partie cristallisable, une chaîne pendante cristallisable ou une séquence cristallisable dans le squelette, et une partie amorphe dans le squelette, qui présente, en outre, une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe. Le polymère semi-cristallin est dans ce cas un copolymère séquencé par exemple du type dibloc, tribloc ou multibloc, comportant au moins une séquence cristallisable et au moins une séquence amorphe. Par « séquence », on entend généralement au moins 5 motifs de répétition identiques. La ou les séquences cristallisables sont alors de nature chimique différente de la ou des séquences amorphes.

Le polymère semi-cristallin a une température de fusion supérieure ou égale à 30 °C, notamment allant de 30 °C à 80 °C, de préférence allant de 30 °C à 60 °C. Cette température de fusion est une température de changement d'état du premier ordre.

Cette température de fusion peut être mesurée par toute méthode connue et en particulier à l'aide d'un calorimètre à balayage différentiel (D.S.C).

De façon avantageuse, le ou les polymères semi-cristallins auxquels s'applique l'invention présentent une masse moléculaire moyenne en nombre supérieure ou égale à 1 000. De façon avantageuse, le ou les polymères semi-cristallins de la composition selon l'invention ont une masse moléculaire moyenne en nombre $\overline{M}n$ allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000. Par « *chaîne ou séquence cristallisable* », on entend au sens de l'invention une chaîne ou séquence qui, si elle était seule, passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère. Avantageusement, la "chaîne pendante cristallisable" peut être une chaîne comportant au moins 6 atomes de carbone.

Le polymère semi-cristallin peut être choisi parmi les copolymères séquencés comportant au moins une séquence cristallisable et au moins une séquence amorphe, les homopolymères et les copolymères portant au moins une chaîne latérale cristallisable par motif de répétition et leurs mélanges.

De tels polymères sont décrits, par exemple, dans le document EP 1 396 259.

Selon un mode plus particulier de réalisation de l'invention, le polymère est issu d'un monomère à chaîne cristallisable choisi parmi les (méth)acrylates d'alkyle saturés en $C_{14}$ à $C_{22}$.

A titre d'exemple particulier de polymère semi-cristallin structurant utilisable dans la composition selon l'invention, on peut citer les produits Intelimer® de la société Landec décrits dans la brochure « Intelimer® polymers », Landec IP22 (Rev. 4-97).

Ces polymères sont sous forme solide à température ambiante (25 °C). Ils sont porteurs de chaînes latérales cristallisables.

Le ou les polymère(s) semi-cristallin peu(ven)t être présent(s) en une teneur allant de 0,1 à 30 % en poids par rapport au poids total de la composition.

La composition selon l'invention peut aussi comprendre, en outre, au moins un gélifiant lipophile.

## Gélifiants lipophiles

Les gélifiants utilisables dans les compositions selon l'invention peuvent être des gélifiants lipophiles, organiques ou minéraux, polymériques ou moléculaires.

Comme gélifiant lipophile minéral, on peut citer les argiles, éventuellement modifiées, comme les hectorites modifiées par un chlorure d'ammonium d'acide gras en $C_{10}$ à $C_{22}$, comme l'hectorite modifiée par du chlorure de di-stéaryl di-méthyl ammonium telle que, par exemple, celle commercialisée sous la dénomination de Bentone 38V® par la société ELEMENTIS.

On peut également citer la silice pyrogénée, éventuellement hydrophobe traitée en surface, dont la taille des particules est inférieure à 1 $\mu$m. En effet, il est possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :

- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (6ème édition, 1995). Elles sont par exemple commercialisées sous les références Aerosil R812® par la société DEGUSSA, CAB-O-SIL TS-530® par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (6ème édition, 1995). Elles sont par exemple commercialisées sous les références Aerosil R972®, et Aerosil R974® par la société DEGUSSA, CAB-O-SIL TS-610® et CAB-O-SIL TS-720® par la société CABOT.

La silice pyrogénée hydrophobe présente en particulier une taille de particules pouvant être nanométrique à micrométrique, par exemple allant d'environ de 5 à 200 nm.

On peut également utiliser des gélifiants organique moléculaires non polymériques, également appelés organogélateurs, associés à une phase grasse liquide (qui peut être la phase grasse liquide de la composition selon l'invention), qui sont des composés dont les molécules sont capables d'établir entre elles des interactions physiques conduisant à une auto-agrégation des molécules avec formation d'un réseau supra-moléculaire 3D qui est responsable de la gélification de la phase grasse liquide.

Le réseau supra-moléculaire peut résulter de la formation d'un réseau de fibrilles (dues aux empilements ou agrégations de molécules d'organogélateur), immobilisant les molécules de la phase grasse liquide.

L'aptitude à former ce réseau de fibrilles, et donc à gélifier, dépend de la nature (ou classe chimique) de l'organogélateur, de la nature des substituants portés par ses molécules pour une classe chimique donnée et de la nature de la phase grasse liquide.

Les interactions physiques sont diverses mais excluent la co-cristallisation. Ces interactions physiques sont en particulier des interactions du type interactions hydrogènes auto-complémentaires, interactions $\pi$ entre cycles insaturés, interactions dipolaires, liaisons de coordination avec des dérivés organométalliques et leurs associations. En général, chaque molécule d'un organogélateur peut établir plusieurs types d'interactions physiques avec une molécule voisine. Aussi, avantageusement, les molécules des organogélateurs conformes à l'invention comportent au moins un groupement capable d'établir des liaisons hydrogènes et mieux au moins deux groupements capables d'établir des liaisons hydrogène, au moins un cycle aromatique et mieux aux moins deux cycles aromatiques, au moins une ou plusieurs liaisons à insaturation éthylénique et/ou au moins un ou plusieurs carbones asymétriques. De préférence, les groupements capables de faire des liaisons hydrogènes sont choisis parmi les groupements hydroxyle, carbonyle, amine, acide carboxylique, amide, urée, benzyle et leurs associations.

Le ou les organogélateurs sont solubles dans la phase grasse liquide après chauffage jusqu'à obtention d'une phase liquide homogène transparente. Ils peuvent être solides ou liquides à température ambiante (25 °C) et pression atmosphérique.

Le ou les organogélateurs moléculaires utilisables dans la composition selon l'invention sont notamment ceux décrits dans le document « Specialist Surfactants », édité par D. Robb de 1997, p.209-263, chapitre 8 de P. Terech, les demandes européennes EP-A-1 068 854 et EP-A-1 086 945 ou encore dans la demande WO-A-02/47031.

On peut notamment citer parmi ces organogélateurs, les amides d'acides carboxyliques en particulier les acides tri-carboxyliques comme les cyclohexanetricarboxamides (voir la demande EP-A-1 068 854), les diamides ayant des chaînes hydrocarbonées contenant chacune de 1 à 22 atomes de carbone, par exemple de 6 à 18 atomes de carbone, lesdites chaînes étant non substituées ou substituées avec au moins un substituant choisi parmi les groupes ester, urée et fluoro (voir la demande EP-A-1 086 945) et notamment les diamides résultant de la réaction du diaminocyclohexane, en particulier du diaminocyclohexane sous forme trans, et d'un chlorure d'acide comme par exemple le N,N'-bis (dodécanoyl)-1,2-diaminocyclohexane, les amides de N-acylamino acides comme les diamides résultant de l'action d'un N-acylamino acide avec des amines comportant de 1 à 22 atomes de carbone, comme par exemple ceux décrits dans le document WO 93/23008 et notamment les amides de l'acide N-acylglutamique où le groupe acyle représente une chaîne alkyle en $C_8$ à $C_{22}$ tels que le dibutylamide de l'acide N-Lauroyl-L-glutamique, fabriqué ou commercialisé par la société Ajinomoto sous la dénomination GP-1 et leurs mélanges.

Les gélifiants lipophiles organiques polymériques sont par exemple :

- les organopolysiloxanes élastomériques partiellement ou totalement réticulés, de structure tridimensionnelle, comme ceux commercialisés sous les dénominations de KSG6®, KSG16® et de KSG18® par la société SHIN-ETSU, de Trefil E-505C® et Trefil E-506C® par la société DOW-CORNING, de Gransil SR-CYC®, SR DMF10®, SR-DC556®, SR 5CYC gel®, SR DMF 10 gel® et de SR DC 556 gel® par la société GRANT INDUSTRIES, de SF 1204® et de JK 113® par la société GENERAL ELECTRIC ;
- l'éthylcellulose comme celle vendue sous la dénomination Ethocel® par la société DOW CHEMICAL ;
- les polycondensats de type polyamide résultant de la condensation entre ($\alpha$) au moins un acide choisi parmi les acides dicarboxyliques comprenant au moins 32 atomes de carbone tels que les acides gras dimères et ($\beta$) un alkylène diamine et en particulier l'éthylène diamine, dans lequel le polymère polyamide comprend au moins un groupe acide carboxylique terminal estérifié ou amidifié avec au moins un mono alcool ou une mono amine comprenant de 12 à 30 atomes de carbone, linéaires et saturés, et en particulier, les copolymères d'éthylène diamine/dilinoléate de stéaryle, tel que celui commercialisé sous la dénomination Uniclear 100 VG® par la société ARIZONA CHEMICAL ;
- les polyamides siliconés du type polyorganosiloxane tels que ceux décrits dans les documents US-A-5,874,069, US-A-5,919,441, US-A-6,051,216 et US-A-5,981,680 comme par exemple ceux commercialisés sous la référence

Dow Corning 2-8179 Gellant® par la société DOW CORNING;

- les galactommananes comportant de un à six, et en particulier de deux à quatre, groupes hydroxyle par ose, substitués par une chaîne alkyle saturée ou non, comme la gomme de guar alkylée par des chaînes alkyle en $C_1$ à $C_6$, et en particulier en $C_1$ à $C_3$ et leurs mélanges ;
- les copolymères séquencés, éventuellement hydrogénés, de type « dibloc », « tribloc » ou « radial », en particulier à blocs styrène et à blocs éthylène/alkylène en $C_3$-$C_4$. Comme copolymère dibloc, de préférence hydrogéné, on peut citer les copolymères de styrène-éthylène/propylène, les copolymères de styrène-éthylène/butadiène. Des polymères diblocs sont notamment vendus sous la dénomination Kraton® G1701E par la société Kraton Polymers.

Comme copolymère tribloc, de préférence hydrogéné, on peut citer les copolymères de styrène-éthylène/propylène-styrène, les copolymères de styrène-éthylène/butadiène-styrène, les copolymères de styrène-isoprène-styrène, les copolymères de styrène-butadiène-styrène. Des polymères triblocs sont notamment vendus sous les dénominations Kraton® G1650, Kraton® G1652, Kraton® D1101, Kraton® D1102, e Kraton D1160 par la société Kraton Polymers.

On peut également utiliser un mélange de copolymère hydrogéné tribloc styrène-butylène/éthylène-styrène et de polymère sous forme d'étoile, hydrogéné d'éthylène-propylène-styrène, un tel mélange étant notamment dans l'isododécane. De tels mélanges sont par exemple vendus par la société PENRECO sous les dénominations commerciales VERSAGEL® M5960 et VERSAGEL® M5670.

On peut également citer les copolymères polystyrène/polyisoprène, polystyrène/polybutadiène tels que ceux commercialisés sous la dénomination Luvitol HSB® par la société BASF.

Parmi les gélifiants lipophiles pouvant être utilisés dans les compositions selon l'invention, on peut encore citer les esters de dextrine et d'acide gras, tels que les palmitates de dextrine, notamment tels que ceux commercialisés sous les dénominations Rheopearl TL® ou Rheopearl KL® par la société CHIBA FLOUR.

Le ou les gélifiants(s) lipophile(s) peu(ven)t être présent(s) en une teneur allant de 0,1 à 30 % en poids, par rapport au poids total de la composition.

La composition selon l'invention peut comprendre, en outre, au moins un composé pâteux.

**Composé pâteux**

Par « pâteux » au sens de la présente invention, on entend un composé gras lipophile à changement d'état solide/liquide réversible et comportant à la température de 23 °C une fraction liquide et une fraction solide. En d'autres termes, la température de fusion commençante du composé pâteux est inférieure à 23 °C. Le composé pâteux est dit « à l'état solide », lorsque l'intégralité de sa masse est sous forme solide et le composé pâteux est dit « à l'état liquide », lorsque l'intégralité de sa masse est sous forme liquide.

La fraction liquide du composé pâteux mesurée à 23 °C représente de 23 à 97 % en poids du composé, de préférence entre 25 et 85 % en poids du composé.

La fraction liquide en poids du composé pâteux à 23 °C est égale au rapport de l'enthalpie de fusion consommée à 23 °C sur l'enthalpie de fusion du composé pâteux.

L'enthalpie de fusion du composé pâteux est la quantité d'énergie nécessaire, consommée par le composé, pour passer de l'état solide à l'état liquide. Elle est exprimée en J/g.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10 °C par minute, selon la norme ISO 11357-3:1999.

L'enthalpie de fusion consommée à 23 °C est la quantité d'énergie absorbée par l'échantillon pour passer de l'état solide à l'état qu'il présente à 23 °C constitué d'une fraction liquide et d'une fraction solide.

La fraction liquide du composé pâteux mesurée à 32 °C représente de préférence de 40 à 100 % en poids du composé, de préférence de 50 à 100 % en poids du composé. Lorsque la fraction liquide du composé pâteux mesurée à 32 °C est égale à 100 %, la température de la fin de la plage de fusion du composé pâteux est inférieure ou égale à 32 °C.

La fraction liquide du composé pâteux mesurée à 32 °C est égale au rapport de l'enthalpie de fusion consommée à 32 °C sur l'enthalpie de fusion du composé pâteux. L'enthalpie de fusion consommée à 32 °C est calculée de la même façon que l'enthalpie de fusion consommée à 23 °C.

Le composé pâteux est de préférence choisi parmi les composés synthétiques et les composés d'origine végétale. Un composé pâteux peut être obtenu par synthèse à partir de produits de départ d'origine végétale.

Le composé pâteux est avantageusement choisi parmi :

- la lanoline et ses dérivés,
- les composés siliconés, polymères ou non,
- les composés fluorés, polymères ou non,
- les polymères vinyliques, notamment:

- les homopolymères d'oléfines,
- les copolymères d'oléfines,
- les homopolymères et copolymères de diènes hydrogénés,
- les oligomères linéaires ou ramifiés, homo ou copolymères de (méth)acrylates d'alkyles ayant de préférence un groupement alkyle en $C_8$-$C_{30}$,
- les oligomères homo et copolymères d'esters vinyliques ayant des groupements alkyles en $C_8$-$C_{30}$,
- les oligomères homo et copolymères de vinyléthers ayant des groupements alkyles en $C_8$-$C_{30}$,
- les polyéthers liposolubles résultant de la polyéthérification entre un ou plusieurs diols en $C_2$-$C_{100}$, de préférence en $C_2$-$C_{50}$,
- les esters et les polyesters,
- et leurs mélanges.

Le composé pâteux est de préférence un polymère, notamment hydrocarboné.

Un composé pâteux siliconé et fluoré préféré est le polyméthyl trifluoropropryl méthylalkyl diméthylsiloxane, fabriqué sous la dénomination X22-1088 par SHIN ETSU.

Lorsque le composé pâteux est un polymère siliconé et/ou fluoré, la composition comprend avantageusement un agent compatibilisant tel que des esters à courte chaîne comme le néopentanoate d'isodécyle.

Parmi les polyéthers liposolubles, on préfère en particulier les copolymères d'éthylène-oxyde et/ou de propylène-oxyde avec des alkylènes-oxydes à longue chaîne en $C_6$-$C_{30}$, de préférence encore tels que le rapport pondéral de l'éthylène-oxyde et/ou de propylène-oxyde avec alkylènes-oxydes dans le copolymère est de (5/95) à (70/30). Dans cette famille, on citera notamment les copolymères tels que les alkylènes-oxydes à longue chaîne qui sont disposés en blocs ayant un poids moléculaire moyen de 1000 à 10000, par exemple un copolymère bloc de polyoxyethylène/polydodécyle glycol tel que les éthers de dodécanediol (22 mol) et de polyéthylène glycol (45 OE) commercialisés sous la marque ELFACOS ST9® par Akzo Nobel.

Parmi les esters, on préfère notamment :

- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide isostéarique et l'acide 12-hydroxystéarique, à l'image notamment de ceux commercialisé sous la marque Softisan 649® par la société Sasol :
- les esters de phytostérol,
- les esters de pentaérythritol
- les esters formés à partir
    - d'au moins un alcool, l'un au moins des alcools étant un alcool de Guerbet et
    - d'un dimère diacide formé à partir d'au moins un acide gras insaturé, comme l'ester de dimère d'acides gras de tallol comprenant 36 atomes de carbone et d'un mélange i) d'alcools de Guerbet comprenant 32 atomes de carbone et ii) d'alcool béhénylique; l'ester de dimère d'acide linoléique et d'un mélange de deux alcools de Guerbet, le 2-tétradécyl-octadécanol (32 atomes de carbone) et le 2-hexadécyl-eicosanol (36 atomes de carbone).
- les polyesters non réticulés résultant de la polycondensation entre un acide dicarboxylique ou un polyacide carboxylique linéaire ou ramifié en $C_4$-$C_{50}$ et un diol ou un polyol en $C_2$-$C_{50}$,
- les polyesters qui résultent de l'estérification, par un acide polycarboxylique d'un ester d'acide hydroxy carboxylique aliphatique comme le Risocast DA-L® et le Risocast DA-H® commercialisés par la société japonaise KOKYU ALCOHOL KOGYO, qui sont des esters résultant de la réaction d'estérification de l'huile de ricin hydrogénée avec l'acide dilinoléïque ou l'acide isostéarique,
- les esters aliphatiques d'ester résultant de l'estérification d'un ester d'acide hydroxycarboxylique aliphatique par un acide carboxylique aliphatique; (Salacos HCIS (V)-L commercialisé par la société Nishing Oil),

L'acide carboxylique aliphatique comprend de 4 à 30 et de préférence de 8 à 30 atomes de carbone. Il est de préférence choisi parmi l'acide héxanoïque, l'acide heptanoïque, l'acide octanoïque, l'acide éthyl-2 héxanoïque, l'acide nonanoïque, l'acide décanoïque, l'acide undécanoïque, acide dodécanoïque, l'acide tridécanoïque, l'acide tétradécanoïque, l'acide pentadécanoïque, l'acide héxadécanoïque, l'acide héxyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.

L'acide carboxylique aliphatique est de préférence ramifié.

L'ester d'acide hydroxy carboxylique aliphatique est avantageusement issu d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle. L'ester d'acide hydroxy carboxylique aliphatique est choisi parmi :

a) les esters partiels ou totaux d'acides monocarboxyliques aliphatiques mono hydroxylés linéaires, saturés ;
b) les esters partiels ou totaux d'acides monocarboxyliques aliphatiques mono hydroxylés insaturés ;
c) les esters partiels ou totaux de polyacides carboxyliques aliphatiques mono hydroxylés saturés ;
d) les esters partiels ou totaux de polyacides carboxyliques aliphatiques poly hydroxylés saturés ;
e) les esters partiels ou totaux de polyols aliphatiques en $C_2$ à $C_{16}$ ayant réagi avec un mono ou un poly acide carboxylique aliphatique mono ou poly hydroxylé, et leurs mélanges.

Les esters aliphatiques d'ester sont avantageusement choisis parmi :

- l'ester résultant de la réaction d'estérification de l'huile de ricin hydrogénée avec l'acide isostéarique dans les proportions 1 pour 1 (1/1) ou monoisostéarate d'huile de ricin hydrogénée,
- l'ester résultant de la réaction d'estérification de l'huile de ricin hydrogénée avec l'acide isostéarique dans les proportions 1 pour 2 (1/2) ou le diisostéarate d'huile de ricin hydrogénée,
- l'ester résultant de la réaction d'estérification de l'huile de ricin hydrogénée avec l'acide isostéarique dans les proportions 1 pour 3 (1/3) ou triisostéarate d'huile de ricin hydrogénée,
- et leurs mélanges.

De préférence, le composé pâteux est choisi parmi les composés d'origine végétale.
Parmi ceux-ci, on peut notamment citer l'huile de jojoba isomérisée telle que l'huile de jojoba partiellement hydrogénée isomérisée trans fabriquée ou commercialisée par la société Desert Whale sous la référence commerciale Iso-Jojoba-50®, la cire d'orange comme, par exemple, celle qui est commercialisée sous la référence Orange Peel Wax® par la société Koster Keunen, le beurre de karité, l'huile d'olive partiellement hydrogénée comme, par exemple, le composé commercialisé sous la référence Beurrolive® par la société Soliance, le beurre de cacao, l'huile de mangue comme, par exemple, la Lipex® 302 de la société Aarhuskarlshamn.
Le composé pâteux peut représenter de 0,5 à 20 %, mieux 1 à 15 % en poids par rapport au poids total de la composition.

**<u>Phase grasse liquide</u>**

La composition selon l'invention peut comprendre une phase grasse liquide.
Par « phase grasse liquide », au sens de la demande, on entend une phase grasse liquide à température ambiante (25 °C) et pression atmosphérique (760 mm de Hg), composée d'un ou plusieurs corps gras, non aqueux, liquides à température ambiante et compatibles entre eux. On appelle aussi ces corps gras des huiles.
L'huile peut être choisie parmi les huiles volatiles, les huiles non volatiles et leurs mélanges.
Par « huile volatile », on entend au sens de l'invention une huile susceptible de s'évaporer au contact de la peau ou de la fibre kératinique en moins d'une heure, à température ambiante et pression atmosphérique. La ou les huiles volatiles de l'invention sont des solvants organiques volatils et/ou des huiles cosmétiques volatiles, liquides à température ambiante et ayant une pression de vapeur non nulle à température ambiante et pression atmosphérique, allant en particulier de 0,13 Pa à 40 000 Pa (soit de $10^{-3}$ à 300 mm de Hg), en particulier allant de 1,3 Pa à 13 000 Pa (soit de 0,01 à 100 mm de Hg), et plus particulièrement allant de 1,3 Pa à 1300 Pa (soit de 0,01 à 10 mm de Hg).
Par « huile non volatile », à contrario, on entend une huile restant sur la peau ou la fibre kératinique à température ambiante et pression atmosphérique au moins plus d'une heure et ayant notamment une pression de vapeur inférieure à $10^{-3}$ mm de Hg, soit inférieure à 0,13 Pa.
Ces huiles peuvent être des huiles hydrocarbonées, des huiles siliconées, des huiles fluorées, ou leurs mélanges.
On entend par « huile hydrocarbonée », une huile contenant principalement des atomes d'hydrogène et de carbone et éventuellement des atomes d'oxygène, d'azote, de soufre et/ou de phosphore.
A titre d'exemple d'huile utilisable dans l'invention, on peut citer :

- les huiles hydrocarbonées d'origine animale telles que le perhydrosqualène ;

- les huiles hydrocarbonées végétales telles que les triglycérides liquides d'acides gras de 4 à 24 atomes de carbone comme les triglycérides des acides heptanoïque ou octanoïque ou encore les huiles de tournesol, de maïs, de soja,

de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, de beurre de karité ;

- les hydrocarbures linéaires ou ramifiés, d'origine minérale ou synthétique tels que les huiles de paraffine et leurs dérivés, la vaseline, les polydécènes, les polybutènes, le polyisobutène hydrogéné tel que le Parleam, les huiles hydrocarbonées ayant de 8 à 16 atomes de carbones, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permetyl®, ou encore les distillats de pétrole, notamment ceux vendus sous la dénomination Shell Solt® par la société SHELL ;
- les esters et les éthers de synthèse notamment d'acides gras comme les huiles de formule $R_1COOR_2$ dans laquelle $R_1$ représente le reste d'un acide gras supérieur comportant de 1 à 40 atomes de carbone et $R_2$ représente une chaîne hydrocarbonée contenant de 1 à 40 atomes de carbone avec $R_1 + R_2 \geq 10$ comme par exemple l'huile de Purcellin, l'isononanoate d'isononyle, le myristate d'isopropyle, le palmitate d'éthyl 2-hexyle, le stéarate d'octyl 2-dodécyle, l'érucate d'octyl 2-dodécyle, l'isostéarate d'isostéaryle, le tridecyl trimellitate ; les esters hydroxylés comme l'isostéaryl lactate, l'octyl hydroxy stéarate, l'hydroxy stéarate d'octyl dodécyle, le diisostéaryl malate, le citrate de triisocétyle, des heptanoates, octanoates, décanoates d'alcools gras ; des esters de polyol comme le dioctanoate de propylène glycol, le diheptanoate de néopentyl glycol, le diisononanoate de diéthylène glycol ; et les esters du pentaérythritol comme le tétra-isostéarate de pentaérythrytyle ;
- des alcools gras ayant de 12 à 26 atomes de carbone comme l'octyl dodécanol, le 2-butyloctanol, le 2-hexyl décanol, le 2-undécyl pentadécanol, l'alcool oléique ;
- les huiles fluorées éventuellement partiellement hydrocarbonées et/ou siliconées;
- les huiles siliconées comme les polydiméthylsiloxanes (PDMS) volatiles ou non, linéaires ou cycliques ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényl triméthicones, les phényl diméthicones, les phényl triméthylsiloxy diphényl siloxanes, les diphényl diméthicones, les diphényl méthyldiphényl trisiloxanes, les 2-phényl éthyl triméthyl-siloxysilicates ;
- leurs mélanges.

De préférence, l'huile a une masse moléculaire supérieure ou égale à 250 g/mol notamment entre 250 et 10000 g/mol, de préférence supérieure ou égale à 300 g/mol, notamment entre 300 et 8000 g/mol et mieux, supérieure ou égale à 400 g/mol, notamment entre 400 et 5000 g/mol.
Cette huile peut être choisie parmi :

- les polybutylènes tels que l'INDOPOL H-100 (de masse molaire ou MM=965 g/mol), l'INDOPOL H-300 (MM=1340 g/mol), l'INDOPOL H-1500 (MM=2160g/mol) commercialisés ou fabriqués par la société AMOCO ;
- les polyisobutylènes hydrogénés tels que le PANALANE H-300 E commercialisé ou fabriqué par la société AMOCO (M =1340 g/mol), le VISEAL 20000 commercialisé ou fabriqué par la société SYNTEAL (MM=6000 g/mol), le REWOPAL PIB 1000 commercialisé ou fabriqué par la société WITCO (MM=1000 g/mol) ;
- Les polydécènes et les polydécènes hydrogénés tels que : le PURESYN 10 (MM=723 g/mol), le PURESYN 150 (MM=9200 g/mol) commercialisé ou fabriqués par la société MOBIL CHEMICALS,
- les esters tels que
  - les esters d'acides gras linéaires ayant un nombre total de carbone allant de 30 à 70 comme le tétrapélargonate de pentaérythrityle (MM=697,05 g/mol),
  - les esters hydroxylés tels que le malate de diisostéaryle (MM= 639 g/mol),
  - les esters aromatiques tels que le tridecyl trimellitate (MM=757,19 g/mol),
  - les esters d'alcool gras ou d'acides gras ramifiés en C24-C28 tels que ceux décrits dans la demande EP-A-0 955 039, et notamment le citrate de triisocétyle (MM= 865 g/mol), le tétraisononanoate de pentaérythrityle (MM=697,05g/mol), le triisostéarate de glycéryle (MM=891,51 g/mol), le tri décyl-2 tétradécanoate de glycéryle (MM=1143,98 g/mol), le tétraisostéarate de pentaérythrityle (MM=1202,02 g/mol), le tétraisostéarate de polyglycéryle -2 (MM=1232,04 g/mol) ou encore le tétra décyl-2 tétradécanoate de pentaérythrityle (MM=1538,66 g/mol),
- les huiles d'origine végétale telles que l'huile de sésame (820,6 g/mol),
- et leurs mélanges.

Selon un mode de réalisation particulier, la composition est exempte de phase grasse liquide. Par « exempte de phase grasse liquide », on entend dans le cadre de la présente invention que la composition comprend moins de 5 %, de préférence moins de 1 % de phase grasse liquide.

La composition selon l'invention peut comprendre, de plus, au moins un polymère filmogène, notamment choisi parmi les polymères filmogènes radicalaires, les polycondensats filmogènes, les polymères filmogènes d'origine naturelle et leurs mélanges.

**Polymères filmogènes**

Dans la présente invention, on entend par « polymère filmogène », un polymère apte à former à lui seul ou en présence d'un agent auxiliaire de filmification, un film macroscopiquement continu et adhérent sur les cils, et de préférence un film cohésif, et mieux encore un film dont la cohésion et les propriétés mécaniques sont telles que ledit film peut être isolable et manipulable isolément, par exemple lorsque ledit film est réalisé par coulage sur une surface anti-adhérente comme une surface téflonnée ou siliconée.

Parmi les polymères filmogènes utilisables dans la composition de la présente invention, on peut citer les polymères synthétiques, de type radicalaire ou de type polycondensat, les polymères d'origine naturelle, et leurs mélanges.

Le ou les polymère(s) filmogène(s) peuvent être présent(s) dans la composition selon l'invention en une teneur en extrait sec allant de 0,1 % à 30 % en poids, par rapport au poids total d'extrait sec de ladite composition, de préférence de 0,5 % à 20 % en poids, et mieux de 1 % à 15 % en poids.

La composition selon l'invention peut comprendre, en outre, au moins un polymère filmogène radicalaire.

*Polymères filmogènes radicalaires*

Par « *polymère filmogène radicalaire* », on entend un polymère obtenu par polymérisation de monomères à insaturation notamment éthylénique, chaque monomère étant susceptible de s'homopolymériser (contrairement aux polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styréniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

La composition selon l'invention peut comprendre également au moins un polycondensat filmogène.

*Polycondensats filmogènes*

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement $-SO_3M$, avec M représentant un atome d'hydrogène, un ion ammonium $NH_4^+$ ou un ion métallique, comme par exemple un ion $Na^+$, $Li^+$, $K+$, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement $-SO_3M$.

Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement $-SO_3M$ : l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique.

La composition selon l'invention peut comprendre, en outre, au moins un polymère filmogène d'origine naturelle.

*Polymère filmogène d'origine naturelle*

Les polymères filmogènes d'origine naturelle, éventuellement modifiés, peuvent être choisis parmi la résine shellac, la gomme de sandaraque, les dammars, les élémis, les copals, les polymères cellulosiques, et leurs mélanges.

Selon un mode de réalisation de la composition selon l'invention, le polymère filmogène peut être un polymère hydrosoluble et peut être présent dans la phase aqueuse de la composition; le polymère est donc solubilisé dans la phase aqueuse de la composition.

Selon une autre variante de réalisation de la composition selon l'invention, le polymère filmogène peut être un polymère solubilisé dans une phase grasse liquide comprenant des huiles ou solvants organiques tels que ceux décrits précédemment (on dit alors que le polymère filmogène est un polymère liposoluble). De préférence, la phase grasse liquide comprend une huile volatile, éventuellement en mélange avec une huile non volatile, les huiles pouvant être choisies parmi les huiles citées précédemment.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle

du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2 232 303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de « MDTQ », la résine étant décrite en fonction des différentes unités monomériques siloxane qu'elle comprend, chacune des lettres « MDTQ » caractérisant un type d'unité.

A titre d'exemples de résines polyméthylsilsesquioxanes commercialement disponibles, on peut citer celles qui sont commercialisés :

- par la société Wacker sous la référence Resin MK tels que la Belsil PMS MK,
- par la société SHIN-ETSU sous les références KR-220L.

Comme résines siloxysilicates, on peut citer les résines triméthylsiloxysilicate (TMS) telles que celles commercialisées sous la référence SR1000 par la société Général Electric ou sous la référence TMS 803 par la société Wacker. On peut encore citer les résines timéthylsiloxysilicate commercialisées dans un solvant tel que la cyclomethicone, vendues sous la dénomination «KF-7312J» par la société Shin-Etsu, « DC 749 », « DC 593 » par la société Dow Corning.

On peut aussi citer des copolymères de résines de silicone telles que celles citées ci-dessus avec des polydiméthylsiloxanes, comme les copolymères adhésifs sensibles à la pression commercialisés par la société Dow Corning sous la référence BIO-PSA® et décrits dans le document US 5,162,410 ou encore les copolymères siliconés issus de la réaction d'un résine de silicone, telle que celles décrites plus haut, et d'un diorganosiloxane tels que décrits dans le document WO 2004/073626.

Selon un mode de réalisation de l'invention, le polymère filmogène est un polymère éthylénique séquencé linéaire filmogène, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre.

De tels polymères sont décrits par exemple dans les documents EP 1 411 069 ou WO 04/028488.

Le polymère filmogène peut être également présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations Neocryl XK-90®, Neocryl A-1070®, Neocryl A-1090®, Neocryl BT-62®, Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO; Syntran 5760® par la société Interpolymer, Allianz OPT par la société ROHM & HAAS, les dispersions aqueuses de polymères acryliques ou styrène/acrylique vendues sous le nom de marque JONCRYL® par la société JOHNSON POLYMER ou encore les dispersions aqueuses de polyuréthane vendues sous les dénominations Neorez R-981® et Neorez R-974® par la société AVECIA-NEORESINS, les Avalure UR-405®, Avalure UR-410®, Avalure UR-425®, Avalure UR-450®, Sancure 875®, Sancure 861®, Sancure 878® et Sancure 2060® par la société GOODRICH, Impranil 85® par la société BAYER, Aquamere H-1511® par la société HYDROMER ; les sulfopolyesters vendus sous le nom de marque Eastman AQ® par la société Eastman Chemical Products, les dispersions vinyliques comme le Mexomère PAM® de la société CHIMEX et leurs mélanges.

Comme exemples de dispersions non aqueuses de polymère filmogène, on peut citer les dispersions acryliques dans l'isododécane comme le Mexomère PAP® de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrites notamment dans le document WO 04/055081.

La composition selon l'invention peut comprendre en outre au moins un agent plastifiant favorisant la formation d'un film avec le polymère filmogène. Un tel agent plastifiant peut être choisi parmi tous les composés connus de l'homme du métier comme étant susceptibles de remplir la fonction recherchée.

La composition selon l'invention peut également comprendre, au moins une matière colorante.

## Matière(s) colorante(s)

La composition selon l'invention peut comprendre au moins une matière colorante choisie parmi les matières pulvérulentes, les colorants liposolubles et les colorants hydrosolubles.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.

La ou les matière(s) colorante(s) peuvent être présentes dans la composition selon l'invention en une teneur allant de 0,01 à 30 % en poids par rapport au poids total de ladite composition.

La composition selon l'invention peut, en outre, comprendre au moins une charge.

## Charge(s)

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon commercialisé sous la dénomination Orgasol® par la société ARKEMA FRANCE, de poly-β-alanine et de polyéthylène, les poudres de polymères de tétrafluoroéthylène comme le Téflon®, la lauroyl-lysine, l'amidon, le nitrure de bore, les microsphères creuses polymériques expansées telles que celles de chlorure de polyvinylidène/acrylonitrile comme celles commercialisées sous la dénomination d'Expancel® par la société Nobel Industrie, les poudres acryliques telles que celles commercialisées sous la dénomination Polytrap® par la société Dow Corning, les particules de polyméthacrylate de méthyle et les microbilles de résine de silicone comme celles commercialisées sous la dénomination Tospearls® par la société Toshiba, le carbonate de calcium précipité, le carbonate et l'hydro-carbonate de magnésium, l'hydroxyapatite, les microsphères de silice creuses comme celles commercialisées sous la dénomination Silica Beads® par la société MAPRECOS, les microcapsules de verre ou de céramique, les savons métalliques dérivés d'acides organiques carboxyliques ayant de 8 à 22

atomes de carbone, et en particulier de 12 à 18 atomes de carbone, par exemple le stéarate de zinc, de magnésium ou de lithium, le laurate de zinc, le myristate de magnésium.

On peut également utiliser un composé susceptibles de gonfler à la chaleur et notamment des particules thermo-expansibles, telles que les microsphères non expansées de copolymère de chlorure de vinylidène/d'acrylonitrile/méthacrylate de méthyle ou de copolymère d'homopolymère d'acrylonitrile comme, par exemple, celles commercialisées respectivement sous les références Expancel® 820DU40 et Expancel® 007WU par la Société AKZO NOBEL.

La ou les charge(s) peuvent représenter de 0,1 à 25 % en poids, en particulier de 1 à 20 % en poids, par rapport au poids total de la composition.

La ou les matière(s) colorante(s) et/ou la ou les charge(s) peuvent, en outre, être présents sous forme de « pâte particulaire ».

La composition selon l'invention, lorsqu'elle contient des particules solides à température ambiante, est préparée en les introduisant dans la composition sous forme d'une dispersion colloïdale appelée aussi "pâte particulaire", telle que décrite dans la demande WO 02/39961 dont le contenu est incorporé par référence dans la présente demande.

Par dispersion colloïdale ou "pâte particulaire", on entend au sens de l'invention une dispersion colloïdale concentrée de particules enrobées ou non dans un milieu continu, stabilisée à l'aide d'un agent dispersant ou éventuellement sans agent dispersant. Ces particules peuvent être choisies parmi les pigments, les nacres, les charges solides et leurs mélanges. Ces particules peuvent être de toute forme notamment de forme sphérique ou allongée comme des fibres. Elles sont insolubles dans le milieu.

L'agent dispersant sert à protéger les particules dispersées contre leur agglomération ou floculation. La concentration en dispersant généralement utilisée pour stabiliser une dispersion colloïdale est de 0,3 à 5 $mg/m^2$, de préférence de 0,5 à 4 $mg/m^2$, de surface de particules. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en $C_8$ à $C_{20}$ et de polyol comme le glycérol, la diglycérine, tel que le stéarate d'acide poly(12-hydroxystéarique) de poids moléculaire d'environ 750g/mole tel que celui vendu sous le nom de Solsperse® 21000 par la société Avecia, le polygycéryl-2 dipolyhydroxystéarate (nom CTFA) vendu sous la référence Dehymyls® PGPH par la société Henkel ou encore l'acide polyhydroxystéarique tel que celui vendu sous la référence Arlacel® P100 par la société Uniqema et leurs mélanges.

Comme autre dispersant utilisable dans la composition de l'invention, on peut citer les dérivés ammonium quaternaire d'acides gras polycondensés comme le Solsperse® 17 000 vendu par la société Avecia, les mélanges de poly diméthylsiloxane/oxypropylène tels que ceux vendus par la société Dow Corning sous les références DC2-5185, DC2-5225 C.

L'acide polydihydroxystéarique et les esters de l'acide hydroxy-12-stéarique sont de préférence destinés à un milieu hydrocarboné ou fluoré, alors que les mélanges de diméthylsiloxane oxyéthylène/oxypropyléné sont de préférence destinés à un milieu siliconé.

La dispersion colloïdale est une suspension de particules de taille généralement micronique (<10 $\mu$m) dans un milieu continu. La fraction volumique de particules dans une dispersion concentrée est de 20 % à 40 %, de préférence supérieure à 30 %, ce qui correspond à une teneur pondérale pouvant aller jusqu'à 70 % selon la densité des particules.

Les particules dispersées dans le milieu peuvent être constituées de particules minérales ou organiques ou de leurs mélanges tels que ceux décrits ci-après.

Le milieu continu de la pâte peut être quelconque et contenir tout solvant ou corps gras liquide et leurs mélanges. Avantageusement, le milieu liquide de la pâte particulaire est l'un des corps gras liquides ou huiles que l'on souhaite utiliser dans la composition, faisant ainsi partie de la phase grasse liquide.

De façon avantageuse, la « pâte particulaire » ou dispersion colloïdale est une « pâte pigmentaire » contenant une dispersion colloïdale de particules colorées, enrobées ou non. Ces particules colorées sont des pigments, des nacres ou un mélange de pigments et/ou de nacres.

Avantageusement, la dispersion colloïdale représente de 0,5 à 30 % en poids de la composition, mieux de 2 à 20 % et encore mieux de 2 à 15 %.

La composition selon l'invention peut, en outre, comprendre au moins une fibre.

## Fibres

Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses

ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 μm à 10 mm, en particulier de 0,1 mm à 5 mm et plus particulièrement de 0,3 mm à 3,5 mm. Leur section peut être comprise dans un cercle de diamètre allant de 2 nm à 500 μm, en particulier allant de 100 nm à 100 μm et plus particulièrement de 1 μm à 50 μm. Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.

Les fibres utilisables dans la composition de l'invention peuvent être choisies parmi les fibres rigides ou non rigides, elles peuvent être d'origine synthétique ou naturelle, minérales ou organiques.

Par ailleurs, les fibres peuvent être traitées ou non en surface, enrobées ou non, colorées ou non colorées.

A titre de fibres utilisables dans la composition selon l'invention, on peut citer les fibres non rigides telles que les fibres de polyamide (Nylon®) ou les fibres rigides telles que les fibres de polyimide-amide comme celles vendues sous les dénominations KERMEL®, KERMEL TECH® par la société RHODIA ou de poly-(p-phénylène-téréphtalamide) (ou d'aramide) notamment vendues sous la dénomination Kevlar® par la société DUPONT DE NEMOURS.

Les fibres peuvent êtres présentes dans la composition selon l'invention en une teneur allant de 0,01 % à 10 % en poids, par rapport au poids total de la composition, en particulier de 0,1 % à 5 % en poids, et plus particulièrement de 0,3 % à 3 % en poids.

Les compositions selon l'invention peuvent comprendre, en outre, tout additif usuellement utilisé en cosmétique tels que les antioxydants, les conservateurs, les parfums, les neutralisants, les épaississants, les vitamines, les hydratants, les filtres en particulier solaires, les agents de coalescence, les plastifiants, et leurs mélanges.

Bien entendu, l'homme du métier veillera à choisir les éventuels additifs complémentaires et/ou leur quantité de telle manière que les propriétés avantageuses des compositions selon l'invention ne soient pas, ou substantiellement pas, altérées par l'adjonction envisagée.

Les compositions selon la présente invention, à savoir en particulier caractérisée par sa dureté, peuvent être obtenues par « trempe à froid » de compositions pouvant quant à elles être préparées selon différentes méthodes connues de l'homme du métier.

La méthode de « *trempe à froid* »

Cette trempe à froid consiste à traiter la composition obtenue selon les méthodes classiques, notamment sous forme d'émulsion cire-dans-eau, dans des conditions de refroidissement telles que la composition acquiert une dureté conforme à l'invention, par exemple par placement de la composition dans une enceinte réfrigérée.

En particulier, la trempe consiste à opérer un refroidissement en une durée comprise entre 15 et 60 minutes à une température comprise entre -30 °C et 10 °C, en particulier entre -28 °C et 0 °C, par exemple par placement au congélateur.

L'air peut être brassé pour obtenir une température homogène dans l'enceinte.

Selon un mode de réalisation, la composition est obtenue selon le procédé comprenant les étapes successives suivantes de :

(i) chauffage des phases grasses et aqueuses conformes à l'invention, à une température comprise entre 80 °C et 100 °C, plus particulièrement entre 85 °C et 100 °C, par exemple à une température de 95 °C,
(ii) mise en émulsion desdites phases à l'aide notamment d'un agitateur Moritz, comme cela est classiquement réalisé pour l'élaboration d'un mascara en vue de l'obtention d'une composition selon l'invention,
(iii) coulage à chaud de la composition obtenue à l'étape précédente (ii) dans un dispositif de conditionnement, puis
(iv) placement au congélateur de l'ensemble, notamment à une température comprise entre -30 °C et 10 °C, par exemple à une température de -28 °C, pendant une durée comprise entre 15 et 60 minutes, par exemple de 45 minutes.

Dans le cadre de la présente invention, l'apport de frigorie conforme à l'invention peut être obtenu par toute méthode connue de l'homme de l'art. Ainsi, différentes méthodes, notamment industrielles, peuvent être mises en oeuvre telles que le passage de l'émulsion dans tunnel à froid, l'application d'air pulsé etc

De manière avantageuse, la composition selon l'invention se présente sous la forme d'un stick, notamment d'un stick de mascara.

En outre, la composition selon l'invention peut être conditionnée dans un dispositif de conditionnement et d'application comprenant au moins :

i. un stick comprenant une composition cosmétique telle que définie précédemment;

ii. un support dudit stick; et

iii. éventuellement au moins un élément d'application, ledit élément d'application pouvant être en outre muni d'organes de séparation et/ou de peignage des fibres kératiniques.

La composition selon la présente invention peut être conditionnée comme cela est proposé dans la demande EP 1 913 835. Comme cela est illustré sur la figure 1, le dispositif 1 de conditionnement et d'application d'une composition P cosmétique selon l'invention sous forme d'un stick peut comporter un plateau sur lequel repose ledit stick, une cheminée 2 formant un logement pour ledit stick de composition P, ledit plateau se trouvant dans ladite cheminée, et un moyen d'entraînement 5 pour le déplacement en translation du plateau dans la cheminée, ledit déplacement du plateau permettant la distribution de ladite composition via une ouverture de distribution 9 à l'extrémité supérieure de la cheminée.

La cheminée est dressée sur une embase 3 présentant un fond 8 et une paroi latérale 7.

L'ouverture de distribution est bordée d'organes de séparation/peignage 6, qui se présentent avantageusement sous la forme d'une rangée de dents effilées, de structure conique à base circulaire, régulièrement répartis le long de la bordure de l'ouverture de distribution 9. A l'intérieur de la cheminée se trouve ledit stick de composition P reposant sur ledit plateau actionné par un bouton-poussoir 4 qui, par l'intermédiaire d'un moyen d'entraînement 5, permet de déplacer le stick de composition P le long d'un axe X de façon à déplacer ledit stick au-delà de l'extrémité des organes de séparation/peignage 6.

Les organes de séparation et/ou peignage 6 peuvent se présenter notamment sous la forme de dents ou de poils, disposés selon au moins une rangée s'étendant d'un côté au moins de l'ouverture de distribution 9, et faisant saillie par rapport à ladite face du support.

Le dispositif peut présenter une surface d'application au moins en partie convexe disposée sur une face du support, la surface d'application étant formée par une partie au moins de la surface latérale d'un stick de la composition selon l'invention.

Ce dispositif 1 permet d'appliquer la composition selon l'invention sur les cils selon un procédé de revêtement décrit ci-après.

Le procédé de revêtement de fibres kératiniques comprend au moins les étapes consistant à :

- mettre lesdites fibres kératiniques au contact d'une partie au moins de la surface d'un stick comprenant une composition cosmétique telle que définie précédemment; puis
- procéder à un déplacement relatif entre la surface dudit stick et lesdites fibres kératiniques, de manière à provoquer le délitage dudit stick et la formation d'un dépôt d'au moins une couche de ladite composition cosmétique sur lesdites fibres kératiniques.

Comme cela est représenté sur la figure 2, le dispositif 1 représenté sur la figure 1 est particulièrement configuré pour l'application d'un produit pâteux sur les cils. L'utilisation d'un tel dispositif se fait préférentiellement en amenant le dispositif 1 au contact de la base des cils 10, en présentant le grand axe Y sensiblement tangent à la cornée. Le trajet que l'utilisatrice impose au dispositif 1 pour obtenir l'enduction souhaitée de ses cils suit une boucle selon un mouvement dont les composantes sont définies dans un plan perpendiculaire à la cornée et parallèle à l'arête du nez.

Le mouvement se décompose en trois temps, et correspond aux déplacements F1, F2 et F3 schématisés sur la figure 2.

Le procédé peut ensuite se poursuivre avantageusement par les étapes suivantes consistant à :

- rétracter le stick à l'intérieur du dispositif 1, notamment à l'intérieur de la cheminée 2, en deçà des organes de séparation/peignage 6 ; puis
- procéder à la séparation et/ou au peignage des cils 10 selon les mêmes déplacements F1, F2 et F3, que pour l'application de la composition P sur lesdits cils via lesdits organes de séparation/peignage 6.

Les exemples qui suivent sont présentés à titre illustratif et non limitatif de l'invention. Sauf indication contraire, les pourcentages sont exprimés en poids par rapport au poids total de la composition concernée.

Tous les exemples et comparatifs 1 à 31 ci-dessous ont été réalisés selon le protocole suivant :

Les phases grasses et aqueuses sont chauffées à une température de 95 °C, puis émulsionnées à l'aide d'un agitateur Moritz, comme cela est classiquement réalisé pour l'élaboration d'un mascara. L'émulsion obtenue est ensuite coulée à chaud dans un dispositif de conditionnement, puis l'ensemble est placé au congélateur à une température de -28 °C pendant 45 minutes de façon à obtenir à l'issue de ce délai des sticks solide de diamètre 8 mm.

**Exemples et comparatifs 1 à 27**

**Tableau 1 :** Formulation commune pour les exemples 1 à 20, ci-après nommée architecture A.

| Nom usuel | Nom INCI | % |
|---|---|---|
| **cire** | | **20,1** |
| p-hydroxybenzoate de propyle | propylparaben | 0,20 |
| alcool stéarylique oxyéthylène | steareth-2 | 3,33 |
| oxyde de fer noir | CI 77499 | 7,14 |
| Mélange de poly diméthylsiloxane et de silice hydratée | siméthicone | 0,13 |
| p-hydroxybenzoate de méthyle | méthylparaben | 0,15 |
| eau désionisée microbiologique propre | aqua | 58,78 |
| potassium cétyl phosphate | potassium cétyl phosphate | 5,29 |
| hydroxyéthyl cellulose | hydroxyéthylcellulose | 0,89 |
| gomme arabique | acacia | 3,39 |
| phénoxy-2 éthanol | phénoxyethanol | 0,60 |

**Tableau 2 :** Formulation commune pour les exemples 21 à 27, ci-après nommée architecture B

| Nom INCI | (%) |
|---|---|
| Cires | 20,1 |
| Propylparaben | 0,2 |
| Steareth-2 | 3,33 |
| CI77266 (Black 2) (DISTINCTIVE INK BLACK LO AQ de Distinctive Cosmetic Ingredients) | 2 |
| Siméthicone | 0,13 |
| Méthylparaben | 0,23 |
| Glycérine | 5 |
| Eau | 58,94 |
| Potassium cétyl phosphate | 5,29 |
| hydroxyéthylcellulose | 0,89 |
| Acacia | 3,39 |
| Phénoxyéthanol | 0,60 |

La nature des cires des différents exemples et comparatifs 1 à 27 figurant dans le tableau 3 ci-dessous. Est mentionnée également dans le tableau 3, la teneur en cire(s), la teneur en extrait sec (ES) dans la composition, la structure obtenue, ainsi que la dureté en cisaillement d'un stick conformément à la définition selon l'invention pour chacun des exemples et comparatifs 1 à 27.

La teneur théorique en extrait sec de ces exemples et comparatifs est de 41,22 % en poids par rapport au poids total de la composition.

NA dans le tableau 3 signifie que la mesure n'est pas significative.

**Tableau 3**

| Essais | Ex. 1 (invention) Architecture A | Ex. 2 (comparatif) Architecture A | Ex. 3 (invention) Architecture A | Ex. 4 (comparatif) Architecture A | Ex. 5 (comparatif) Architecture A | Ex. 6 (invention) Architecture A |
|---|---|---|---|---|---|---|
| Cires entrant dans la formulation | 3,2 % cire de carnauba<br>4 % cire d'abeille naturelle<br>12,9 % paraffine | 20,1 % cire d'abeille naturelle | 20,1 % cire de carnauba | 20,1 % cire de son de riz | 20,1 % phytowax olive | 20,1 % cire d'abeille synthétique (Cyclochem 326 A d'Evonik Goldschmidt) |
| Extrait sec (% en poids) | 39,1 | 42,6 | 44,2 | 42,4 | 43,0 | 36,6 |
| Structure | stick | pas de stick | Stick* | pas de stick | pas de stick | stick |
| Dureté en cisaillement du stick (en g) | 5,0 ± 0,1 | NA | 10,7 ± 0,8 | NA | NA | 5,3 ± 0,5 |
| Dureté en cisaillement du stick (en g/m) | 625 ± 12,5 | NA | 1337,5 ± 100 | NA | NA | 662,5 ± 62,5 |

* aspect du stick : lisse et dur

**Tableau 3 (suite)**

| Essais | Ex. 7 (comparatif) Architecture A | Ex. 8 (comparatif) Architecture A | Ex. 9 (comparatif) Architecture A | Ex. 10 (comparatif) Architecture A | Ex.11 (comparatif) Architecture A | Ex. 12 (invention) Architecture A |
|---|---|---|---|---|---|---|
| Cires entrant dans la formulation | 20,1 % cire de candelilla | 20,1 % huile de Jojoba hydrogénée | 20,1 % cire de polyéthylène (Performalene 500-L Polyéthylène de New Phase Technologies) | 20,1 % ozokérite | 20,1 % paraffine | 5 % cire de Carnauba + 15,1 % huile d'olive hydrogénée estérifiée avec alcool stéarylique (PHYTOWAX OLIVE 18L57 commercialisé par la société SOPHIM) |
| Extrait sec (% en poids) | 41,2 | 38,3 | 42,6 | 41,0 | 41,7 | 39,6 |
| Structure | pas de stick | pas de stick | pas de stick | Pas de stick | Pas de stick | stick |
| Dureté en cisaillement du stick (en g) | NA | NA | NA | NA | NA | 5,0 ± 0,4 |
| Dureté en cisaillement du stick (en g/m) | NA | NA | NA | NA | NA | 625 ± 50 |

**Tableau 3 (suite)**

| Essais | Ex. 13 (invention) Architecture A | Ex. 14 (invention) Architecture A | Ex. 15 (invention) Architecture A | Ex. 16 (invention) Architecture A | Ex. 17 (invention) Architecture A |
|---|---|---|---|---|---|
| Cires entrant dans la formulation | 5 % Carnauba + 15,1 % cire d'abeille | 5 % Carnauba + 15,1 % paraffine | 5 % Carnauba + 15,1 % Polyéthylène | 5 % Carnauba + 15,1 % ozokérite | 5 % cire d'abeille synthétique +15,1 % ozokérite |
| Extrait sec (% en poids) | 39,4 | 38,5 | 38,9 | 39,3 | 38,0 |
| Structure | stick | stick | stick | stick | stick |
| Dureté en cisaillement du stick (en g) | 4,0 ± 0,1 | 7,0 ± 1,0 | 10,0 ± 1,0 | 9,0 ± 1,9 | 3,0 ± 0,1 |
| Dureté en cisaillement du stick (en g/m) | 500 ± 12,5 | 875 ± 125 | 1250 ± 125 | 1125 ± 237,5 | 375± 12,5 |

**Tableau 3 (suite)**

| Essais | Ex. 18 (invention) Architecture A | Ex. 19 (invention) Architecture A | Ex. 20 (invention) Architecture A | Ex. 21 (comparatif) Architecture B |
|---|---|---|---|---|
| Cires entrant dans la formulation | 1 % cire d'abeille synthétique + 4 % Carnauba + 15,1 % ozokérite | 4 % cire d'abeille synthétique + 1 % Carnauba + 15,1 % ozokérite | 2,5 % cire d'abeille synthétique + 2,5 % Carnauba + 15,1 % ozokérite | 20,1 % cire microcristalline (Base Wax 30540 ou Microwax HW de Paramelt) |
| Extrait sec (% en poids) | 38,4 | 35,6 | 35,9 | 31,4 |
| Structure | stick | stick | stick | pas de stick |
| Dureté en cisaillement du stick (en g) | 8,2 ± 0,8 | 3,7 ± 0,8 | 4,4 ± 0,9 | NA |
| Dureté en cisaillement du stick (en g/m) | 1025± 100 | 462,5± 100 | 550± 112 | NA |

**Tableau 3 (suite)**

| Essais | Ex.22 (comparatif) Architecture B | Ex.23 (comparatif) Architecture B | Ex.24 (comparatif) Architecture B | Ex.25 (comparatif) Architecture B | Ex. 26 (comparatif) Architecture B | Ex. 27 (invention) Architecture B |
|---|---|---|---|---|---|---|
| Cires entrant dans la formulation | 20,1% cire de polyéthylène (Performalene 400 Polyéthylène de New Phase Technologies) | 20,1% $C_{20-40}$ alkyl stéarate (Kester Wax K82H de Koster Keunen) | 20,1 % (hydroxystéaryloxy)stéarate d'alkyle en $C_{20}$-$C_{40}$ (Kester Wax K82P de Koster Keunen) | 20,1 % cire d'abeilles polyglycérolée (Cera Bellina Wax de Koster Keunen) | 1 % Carnauba + 19,1 % ozokérite | 4 % Carnauba + 1 % de cire d'abeille synthétique + 15,1 % ozokérite |
| Extrait Sec (% en poids) | Non mesuré | 40,5 | 30,9 | 30,1 | 31,9 | 33,6 |
| Structure | Pas de stick | Pas de stick | Pas de stick | Pas de stick | Pas de stick | stick |
| Dureté en cisaillement du stick (en g) | NA | NA | NA | NA | NA | 6,2 ± 0,8 |
| Dureté en cisaillement (en g/m) | NA | NA | s | NA | NA | 775 ± 100 |

**Exemple 28**

La composition de l'exemple 28 selon l'invention est présentée dans le tableau 4 ci-dessous. La dureté en cisaillement est exprimée en gramme (et convertie en g/m).

**Tableau 4**

| Ex 28 (invention) | |
|---|---|
| Nom INCI | (%) |
| Cire de Carnauba | 4 |
| Cire d'abeille Synthétique | 1 |
| Ozokérite | 15 |
| Propylparaben | 0,2 |
| Steareth-2 | 5,29 |
| CI77266 (Black 2) (DISTINCTIVE INK BLACK LO AQ de Distinctive Cosmetic Ingredients) | 2 |
| Siméthicone | 0,13 |
| Méthylparaben | 0,23 |
| Glycérine | 5 |
| Eau | 58,94 |
| Potassium cétyl phosphate | 3,33 |
| hydroxyéthylcellulose | 0,89 |

(suite)

| Ex 28 (invention) | |
|---|---|
| Nom INCI | (%) |
| Acacia | 3,39 |
| Phénoxyéthanol | 0,60 |
| Teneur en extrait sec (%) | 33,7 |
| Dureté en cisaillement (en g) | 7,5 g ± 0,9 |
| Dureté en cisaillement (en g/m) | 937,5 ± 112,5 |

On obtient un stick de dureté en cisaillement requise, permettant d'effectuer un dépôt homogène sur les cils, notamment à l'aide d'un dispositif selon les figures annexées.

**Exemple 29**

La composition de l'exemple comparatif 29 est présentée dans le tableau 5 ci-dessous. La dureté en cisaillement est exprimée en grammes et en grammes/m.

**Tableau 5**

| Essais | | Ex 29 (comparatif) |
|---|---|---|
| Nom usuel | Nom INCI | (%) |
| Stéarate de glycéryle (Tegin M Pellets d'Evonik Goldschmidt) | Glyceryl stearate | 2,15 |
| Glycéryle stéarate PEG-30 (Tagat S d'Evonik Goldschmidt) | PEG-30 glyceryl stearate | 5,54 |
| Cire d'abeille blanchie | Cera alba | 7,16 |
| Huile de jojoba hydrogénée | Hydrogenated jojoba oil | 7,16 |
| Cire de Carnauba | Cera Carnauba | 0 |
| *p*-hydroxybenzoate de butyle | butylparaben | 0,12 |
| *p*-hydroxybenzoate d'éthyle | ethylparaben | 0,10 |
| Polybutène (monooléfines/isoparaffines 95/5) (PM = 2060) | polybutene | 1,00 |
| N-oléyl di-hydroshingosine technique | 2-oleamido-1,3-octadecanediol | 0,10 |
| Oxyde de fer noir | CI 77499 | 7,00 |
| Polyquaternium-4 | Polyquaternium-4 | 3,87 |
| Eau désionisée | aqua | 65,31 |
| Glycérol | Glycerin | 0,10 |
| *p*-hydroxybenzoate de méthyle | methylparaben | 0,24 |
| EDTA | Disodium EDTA | 0,10 |
| Hydroxyde de sodium pur | Sodium hydroxide | 0,05 |

Le tableau 6 ci-dessous regroupe l'état d'agrégation, la structure et la dureté en cisaillement d'un stick ayant pour composition la composition de l'Exemple 30 ou 31.

**Tableau 6**

| | Ex 30 | Ex 31 |
|---|---|---|
| Etat d'agrégation | Pas agrégé | Agrégé |

(suite)

| | Ex 30 | Ex 31 |
|---|---|---|
| Teneur en extrait sec (%) | 35,3 | 35,8 |
| Structure | Pas de stick | stick |
| Dureté en cisaillement du stick (en g) | NA | 6,4 ± 0,5 |
| Dureté en cisaillement du stick (en g/m) | NA | 800 ± 62,5 |

Le stick tel qu'obtenu à l'exemple 31 permet d'effectuer un dépôt homogène sur les cils, notamment à l'aide d'un dispositif selon les figures annexées.

**Exemples 32 :** dureté de sticks

Cet exemple a consisté à étudier d'une part l'impact de la cinétique de refroidissement sur la structuration d'un stick et d'autre part la dureté d'un stick en fonction de la durée du refroidissement.

Pour cet exemple, le refroidissement employé est celui du placement de la composition dans une enceinte réfrigérée à -28°C.

Pour ces deux études, un stick a été préparé selon la composition suivante :

| | | Nom INCI | Nom usuel |
|---|---|---|---|
| Phase A | 4,00% | Cera carnauba | Cire de carnauba |
| | 15,00% | Ozokerite | Ozokérite |
| | 1,00% | Synthetic beeswax | Cire d'abeille synthétique |
| | 0,20% | Propylparaben | Propyl paraben |
| | 3,33% | Steareth-2 | Alcool stéarylique oxyéthylène |
| Phase B1 | 0,15% | Methylparaben | Méthyl paraben |
| | 22,72% | Aqua | Eau |
| | 0,13% | Simethicone | Siméthicone |
| | 5,29% | Potassium cetyl phosphate | Potassium cétyl phosphate |
| Phase B2 | 5,00% | Glycerin | Glycérine |
| | 10,00% | CI 77266 (20 %) dispersion in acacia and aqua | Carbon black en dispersion dans un gel de gomme arabique |
| | 0,9% | Hydroxyethylcellulose | Hydroxyéthylcellulose |
| | 2,84% | Acacia | Gomme arabique |
| | 0,6% | Phenoxyethanol | Phénoxyéthanol |
| | Qsp 100 | Aqua | Eau |

Le protocole de préparation du stick à partir de l'émulsion a varié pour mener à bien l'étude dont les objets ont été mentionnés ci-dessus.

Selon le premier volet de l'étude, la cinétique de refroidissement a été modulée.

Ainsi, la première a consisté à laisser le produit refroidir à température ambiante.

Le seconde a consisté à refroidir le produit en le plaçant dans une enceinte refrigérée à - 28°C pendant 45 minutes.

Comme cela est rapporté sur la figure 3 annexée, on observe que suivant la cinétique adoptée on obtient ou non un stick. Cette étude illustre le fait que pour obtenir la structuration requise, permettant d'obtenir une dureté appropriée à l'utilisation envisagée dans le cadre de la présente invention, un apport de frigorie permettant un abaissement de la température de la composition à moins de 4°C, voire moins de 1°C, en moins de 30 minutes doit avantageusement être respecté.

Comme cela est illustré en figure 4 annexée, on observe que pour autant que l'apport de frigorie rappelé ci-dessus est respecté, on obtient des sticks de dureté identique quelle que soit la durée de refroidissement mise en oeuvre.

## Revendications

1. Composition cosmétique de soin et/ou de maquillage des fibres kératiniques sous forme de stick, applicable à sec, (i) se présentant sous la forme d'une émulsion comportant une phase grasse dispersée dans une phase aqueuse, ladite phase grasse comprenant de la cire de Carnauba et/ou de la cire d'abeille synthétique, dans une teneur d'au moins 2 % en poids par rapport au poids total de ladite composition et (ii) présentant une dureté en cisaillement comprise entre 375 g/m et 5000 g/m, ladite dureté en cisaillement étant mesurée à 20 °C sur un stick cylindrique à l'aide d'un fil rigide de diamètre 250 μm en tungstène en faisant avancer le fil relativement au stick à une vitesse de 100 mm/min,
ladite composition présentant une teneur en extrait sec inférieure ou égale à 45% en poids et supérieure ou égale à 25% en poids, par rapport au poids total de la composition,
ladite composition comprenant en outre au moins un agent émulsionnant choisi parmi les tensioactifs anioniques choisis parmi les esters phosphoriques et leurs sels, tels que le potassium cétylphosphate,
ladite composition étant susceptible d'être obtenue, voire obtenue par un procédé comprenant au moins une étape comportant un apport de frigorie permettant un abaissement de la température de la composition à moins de 4°C en moins de 30 minutes,

2. Composition selon la revendication précédente, **caractérisée en ce que** la cire de Carnauba et/ou de cire d'abeille synthétique est comprise dans une teneur d'au moins 3 % en poids, avantageusement au moins 4 % en poids, voire de 5 à 30 % en poids, par rapport au poids total de ladite composition.

3. Composition selon l'une quelconque des revendications précédentes, **caractérisée en ce que** la dureté en cisaillement est comprise entre 500 g/m et 2500 g/m, en particulier entre 750 g/m et 1500 g/m.

4. Composition selon l'une quelconque des revendications précédentes, **caractérisée en ce qu'**elle présente une teneur en extrait sec supérieure ou égale à 30 % en poids et encore plus particulièrement supérieure ou égale à 35 % en poids, par rapport au poids total de ladite composition.

5. Composition selon l'une quelconque des revendications précédentes, **caractérisée en ce qu'**elle comprend en outre au moins une cire additionnelle, autre que la cire d'abeille synthétique et de Carnauba, choisie parmi les cires, solides à température ambiante, éventuellement sous forme de dispersion aqueuse de cire, d'origine animale, végétale, minérale ou de synthèse et leurs mélanges, notamment présentant un point de fusion supérieur ou égal à 45 °C, en particulier supérieur ou égal à 55 °C et/ou possédant un collant supérieur ou égal à 0,7 N.s et une dureté inférieure ou égale à 3,5 MPa.

6. Composition selon la revendication précédente, **caractérisée en ce que** la cire additionnelle est choisie parmi (i) les cires hydrocarbonées telles que la cire d'abeilles naturelle (ou cire d'abeille blanchie), la cire de lanoline, les cires d'insectes de Chine, la cire de riz, la cire de Candellila, la cire d'Ouricurry, la cire d'Alfa, la cire de fibres de liège, la cire de canne à sucre, la cire du Japon et la cire de sumac, la cire de montan, les cires microcristallines, les paraffines, l'ozokérite, les cires de polyéthylène, les cires obtenues par la synthèse de Fisher-Tropsch, les copolymères cireux ainsi que leurs esters, (ii) les cires obtenues par hydrogénation catalytique d'huiles animales ou végétales ayant des chaînes grasses, linéaires ou ramifiées, en $C_8$-$C_{32}$ telles que l'huile de jojoba hydrogénée, l'huile de tournesol hydrogénée, l'huile de ricin hydrogénée, l'huile de coprah hydrogénée et l'huile de lanoline hydrogénée, le tétrastéarate de di-(triméthylol-1,1,1 propane), le tétrabéhénate de di-(triméthylol-1,1,1 propane), (iii) les cires de silicone comme les alkyl ou alkoxy-diméticone ayant de 16 à 45 atomes de carbone, (iv) les cires fluorées, (v) la cire obtenue par hydrogénation d'huile d'olive estérifiée avec l'alcool stéarylique et (vi) les cires obtenues par hydrogénation d'huile de ricin estérifiée avec l'alcool cétylique.

7. Composition selon l'une quelconque des revendications précédentes, **caractérisée en ce qu'**elle comprend en outre de la cire d'ozokérite.

8. Composition selon l'une quelconque des revendications précédentes, **caractérisée en ce qu'**elle présente une teneur totale en cire(s) comprise entre 2 et 35 % en poids, de préférence entre 5 et 30 % en poids, par rapport au poids total de ladite composition.

**9.** Composition selon l'une quelconque des revendications précédentes, **caractérisée en ce qu'**elle comprend en outre au moins un agent émulsionnant choisi parmi les tensioactifs anioniques, non-ioniques, cationiques, amphotériques, les tensioactifs polymériques et leurs mélanges,
les tensioactifs non ioniques étant choisis parmi :

• les tensioactifs non ioniques ayant un HLB supérieur ou égal à 8 à 25 °C, notamment choisis parmi les esters et éthers d'oses, les éthers oxyéthylénés et/ou oxypropylénés de glycérol, les éthers oxyéthylénés et/ou oxypropylénés d'alcools gras, les esters d'acide gras et de polyéthylène glycol, les esters d'acide gras et des éthers de glycérol oxyéthylénés et/ou oxypropylénés, les esters d'acide gras et des éthers de sorbitol oxyéthylénés et/ou oxypropylénés, la diméthicone copolyol, la diméthicone copolyol benzoate, les copolymères d'oxyde propylène et d'oxyde d'éthylène, et leurs mélanges, et
• les tensioactifs non ioniques ayant un HLB inférieur à 8 à 25 °C notamment choisi parmi les esters et éthers d'oses, les éthers oxyéthylénés et/ou oxypropylénés d'alcools gras, les esters d'acides gras et de polyol, les lécithines, le mélange de cyclométhicone/diméthicone copolyol ;

les tensioactifs anioniques étant choisis parmi les sels d'acides gras en $C_{16}$-$C_{30}$, en particulier le stéarate de triéthanolamine, les sels d'acides gras polyoxyéthylénés, les esters phosphoriques et leurs sels, les sulfosuccinates, les alkyléthersulfates, les iséthionates, les acylglutamates, les dérivés de soja, les citrates, les dérivés de proline, les lactylates, les sarcosinates, les sulfonates, les glycinates, et
les tensioactifs amphotériques étant choisis parmi les N-acyl-aminoacides et les oxydes d'amines, les tensioactifs siliconés comme les diméthicone copolyols phosphate.

**10.** Composition selon la revendication 9, **caractérisée en ce qu'**elle comprend au moins un tensioactif anionique additionnel choisi parmi les sels d'acides gras en $C_{16}$-$C_{30}$ notamment ceux dérivant des aminés, comme le stéarate de triéthanolamine ; et éventuellement au moins un tensioactif non ionique choisi parmi les éthers oxyéthylénés et/ou oxypropylénés (pouvant comporter de 1 à 150 groupes oxyéthylénés et/ou oxypropylénés) d'alcools gras (notamment d'alcool en $C_8$-$C_{24}$, et de préférence en $C_{12}$-$C_{18}$) tels que l'éther oxyéthyléné de l'alcool stéarylique à 2 groupes oxyéthylénés ; les esters d'acides gras (notamment d'acide en $C_8$-$C_{24}$, et de préférence en $C_{16}$-$C_{22}$) et de polyol, notamment de glycérol ou de sorbitol, tels que le stéarate de glycéryle; les esters d'acide gras (notamment d'acide en $C_8$-$C_{24}$, et de préférence en $C_{16}$-$C_{22}$) et des éthers de glycérol oxyéthylénés et/ou oxypropylénés (pouvant comporter de 1 à 150 groupes oxyéthylénés et/ou oxypropylénés), comme le stéarate de glycéryle polyéthoxylé à 30 groupes d'oxyde d'éthylène et leurs mélanges.

**11.** Composition selon l'une quelconque des revendications 9 ou 10, **caractérisée en ce qu'**elle comprend de 0,01 à 30 % en poids d'agent émulsionnant, en particulier de 1 à 15 % en poids et encore plus particulièrement de 2 à 10 % en poids par rapport au poids total de ladite composition.

**12.** Procédé de revêtement de fibres kératiniques comprenant au moins les étapes consistant à :

- mettre lesdites fibres kératiniques au contact d'une partie au moins de la surface d'un stick comprenant une composition cosmétique telle que définie selon l'une quelconque des revendications 1 à 11 ; puis
- procéder à un déplacement relatif entre la surface dudit stick et lesdites fibres kératiniques, de manière à provoquer le délitage dudit stick et la formation d'un dépôt d'au moins une couche de ladite composition cosmétique sur lesdites fibres kératiniques.

**13.** Dispositif de conditionnement et d'application d'une composition cosmétique de soin et/ou de maquillage de fibres kératiniques, **caractérisé en ce qu'**il comprend au moins :

• un stick comprenant une composition cosmétique telle que définie selon l'une quelconque des revendications 1 à 11 ;
• un support dudit stick ; et
• éventuellement au moins un élément d'application, ledit élément d'application pouvant être en outre muni d'organes de séparation et/ou de peignage desdites fibres kératiniques.
•

**14.** Composition selon la revendication 1, sous forme de stick destiné au maquillage et/ou au soin des fibres kératiniques, applicable à sec,

(i) se présentant sous la forme d'une émulsion comportant une phase grasse dispersée dans une phase aqueuse, ladite phase grasse comprenant de la cire de Carnauba et/ou de la cire d'abeille synthétique, dans une teneur d'au moins 2 % en poids, de préférence d'au moins 3 % en poids, avantageusement d'au moins 4 % en poids, voire de 5 % à 30 % en poids, par rapport au poids total de ladite composition, ladite composition présentant une teneur en extrait sec inférieure ou égale à 45% en poids et supérieure ou égale à 25% en poids, par rapport au poids total de la composition, ladite composition comprenant en outre au moins un agent émulsionnant choisi parmi les tensioactifs anioniques choisis parmi les esters phosphoriques et leurs sels, tels que le potassium cétylphosphate,
(ii) présentant une dureté en cisaillement comprise entre 375 g/m et 5000 g/m, ladite dureté en cisaillement étant mesurée à 20 °C sur un stick à l'aide d'un fil rigide de diamètre 250 μm en tungstène en faisant avancer le fil relativement au stick à une vitesse de 100 mm/minute,
(iii) ledit stick étant obtenu par un procédé comprenant au moins les étapes suivantes consistant successivement à :

- chauffer ladite émulsion à une température comprise entre 80 °C et 100 C, plus particulièrement entre 85 °C et 100 °C, par exemple à une température de 95°C,
- émulsionner lesdites phases à l'aide notamment d'un agitateur afin d'obtenir une composition de type émulsion à phase continue aqueuse,
- couler à chaud dans un dispositif de conditionnement ladite composition, puis
- procéder à un apport de frigorie permettant un abaissement de la température du stick à moins de 4°C en moins de 30 minutes.

## Patentansprüche

1. Trocken auftragbare, kosmetische Pflege- und/oder Schminkzusammensetzung für Keratinfasern in Stiftform, die (i) in Form einer Emulsion vorliegt, umfassend eine in einer wässrigen Phase dispergierte Fettphase, wobei die Fettphase Carnaubawachs und/oder synthetisches Bienenwachs umfasst, in einem Gehalt von mindestens 2 Gew.-% bezogen auf das Gesamtgewicht der Zusammensetzung, und (ii) eine Scherhärte zwischen 375 g/m und 5000 g/m aufweist, wobei die Scherhärte bei 20 °C an einem zylindrischen Stab mit Hilfe eines starren Wolframdrahts von 250 μm Durchmesser gemessen wird, indem der Draht relativ zu dem Stab mit einer Geschwindigkeit von 100 mm/min vorangetrieben wird,
wobei die Zusammensetzung einen Trockengehalt von kleiner oder gleich 45 Gew.-% und größer oder gleich 25 Gew.-% bezogen auf das Gesamtgewicht der Zusammensetzung aufweist, wobei die Zusammensetzung weiterhin mindestens ein Emulgiermittel umfasst, ausgewählt aus anionischen Tensiden, ausgewählt aus Phosphorsäureestern und ihren Salzen wie Kaliumcetylphosphat,
wobei die Zusammensetzung geeignet ist erhalten zu werden, und zwar durch ein Verfahren erhalten zu werden, umfassend mindestens einen Schritt, umfassend einen Kälteeintrag, der eine Absenkung der Temperatur der Zusammensetzung um bis zu 4 °C in weniger als 30 Minuten ermöglicht.

2. Zusammensetzung nach dem vorhergehenden Anspruch, **dadurch gekennzeichnet, dass** das Carnaubawachs und/oder das synthetische Bienenwachs in einem Gehalt zwischen mindestens 3 Gew.-%, vorteilhafterweise von mindestens 4 Gew.-%, sogar 5 bis 30 Gew.-% bezogen auf das Gesamtgewicht der Zusammensetzung eingeschlossen ist.

3. Zusammensetzung nach einem der vorangehenden Ansprüche, **dadurch gekennzeichnet, dass** die Scherhärte zwischen 500 g/m und 2500 g/m, insbesondere zwischen 750 g/m und 1500 g/m beträgt.

4. Zusammensetzung nach einem der vorangehenden Ansprüche, **dadurch gekennzeichnet, dass** sie einen Gehalt an Trockenextrakt von größer oder gleich 30 Gew.-% und insbesondere größer oder gleich 35 Gew.-% bezogen auf das Gesamtgewicht der Zusammensetzung aufweist.

5. Zusammensetzung nach einem der vorangehenden Ansprüche, **dadurch gekennzeichnet, dass** sie weiterhin mindestens ein zusätzliches Wachs umfasst, das anders ist als synthetisches Bienenwachs und Carnaubawachs, ausgewählt aus bei Umgebungstemperatur festen Wachsen, gegebenenfalls in Form von wässriger Dispersion von Wachs tierischer, pflanzlicher, mineralischer oder synthetischer Herkunft, und ihren Gemischen, die insbesondere einen Schmelzpunkt von größer oder gleich 45 °C, insbesondere größer oder gleich 55 °C aufweisen und/oder eine Klebrigkeit von größer oder gleich 0,7 N.s und eine Härte von kleiner oder gleich 3,5 MPa besitzen.

6. Zusammensetzung nach dem vorhergehenden Anspruch, **dadurch gekennzeichnet, dass** das zusätzliche Wachs ausgewählt ist aus (i) Kohlenwasserstoffwachsen, wie natürliches Bienenwachs (oder gebleichtes Bienenwachs), Lanolinwachs, Chinawachs von Insekten, Reiswachs, Candellilawachs, Ouricouriwachs, Alfawachs, Wachs von Korkfasern, Wachs von Zuckerrohr, Japanwachs, Sumacwachs, Montanwachs, mikrokristalline Wachse, Paraffine, Ozokerit, Polyethylenwachse, durch Fisher-Tropsch-Synthese erhaltene Wachse, wachsartige Copolymere sowie ihre Ester, (ii) Wachsen, die durch katalytische Hydrierung von tierischen oder pflanzlichen Ölen mit linearen oder verzweigten $C_8$-$C_{32}$-Fettsäureketten erhalten werden, wie hydriertes Jojobaöl, hydriertes Sonnenblumenöl, hydriertes Rizinusöl, hydriertes Coprahöl und hydriertes Lanolinöl, Di-(trimethylol-1,1,1-propan)tetrastearat, Di-(trimethylol-1,1,1-propan)tetrabehenat, (iii) Siliconwachsen, wie Alkyl- oder Alkoxydimeticon mit 16 bis 45 Kohlenstoffatomen, (iv) fluorierten Wachsen, (v) Wachs, das durch Hydrierung von verestertem Olivenöl mit Stearylalkohol erhalten wird, und (vi) Wachsen, die durch Hydrierung von verestertem Rizinusöl mit Cetylalkohol erhalten werden.

7. Zusammensetzung nach einem der vorangehenden Ansprüche, **dadurch gekennzeichnet, dass** sie weiterhin Ozokeritwachs umfasst.

8. Zusammensetzung nach einem der vorangehenden Ansprüche, **dadurch gekennzeichnet, dass** sie einen Gesamtgehalt an Wachs(en) zwischen 2 und 35 Gew.-%, vorzugsweise zwischen 5 und 30 Gew.-% bezogen auf das Gesamtgewicht der Zusammensetzung aufweist.

9. Zusammensetzung nach einem der vorangehenden Ansprüche, **dadurch gekennzeichnet, dass** sie weiterhin mindestens ein emulgierendes Mittel umfasst, ausgewählt aus anionischen Tensiden, nicht-ionischen Tensiden, kationischen Tensiden, amphoteren Tensiden, polymeren Tensiden und ihren Gemischen, wobei die ionischen Tenside ausgewählt sind aus:

    • nicht-ionischen Tensiden mit einem HLB von größer oder gleich 8 bis 25 °C, insbesondere ausgewählt aus Estern und Ethern von -osen, Glycerinoxyethylenethern und -oxy-propylenethern, Fettalkoholoxyethylenethern und -oxypropylethern, Estern von Fettsäure und Polyethylenglycol, Estern von Fettsäure und Oxyethylenglycerinethern und/oder Oxypropylenglycerinethern, Estern von Fettsäure und Oxyethylensorbitethern und/oder Oxypropylensorbitethern, Dimethiconcopolyol, Dimethiconcopolyolbenzoat, Copolymeren von Propylenoxid und Ethylenoxid und ihren Gemischen, und
    • nicht-ionischen Tensiden mit einem HLB von kleiner 8 bis 25 °C, insbesondere ausgewählt aus Estern und Ethern von -osen, Fettalkoholoxyethylenethern und/oder -oxy-propylenethern, Estern von Fettsäure und Polyol, Lecithinen, Cyclomethion/DimethiconCopolyol-Gemisch;

    wobei die anionischen Tenside ausgewählt sind aus $C_{16}$-$C_{30}$-Fettsäuresalzen, insbesondere Triethanolaminstearat, Polyoxyethylenfettsäuresalzen, Phosphorsäureestern und ihren Salzen, Sulfosuccinaten, Alkylethersulfaten, Isethionaten, Acylglutamaten, Sojaderivaten, Citraten, Prolinderivaten, Lactylaten, Sarcosinaten, Sulfonaten, Glycinaten, und
    amphoteren Tensiden, die aus N-Acylaminoaciden und Aminoxiden, Silicontensiden, wie Dimethiconcopolyolphosphat, ausgewählt sind.

10. Zusammensetzung nach Anspruch 9, **dadurch gekennzeichnet, dass** sie mindestens ein zusätzliches anionisches Tensid umfasst, ausgewählt aus $C_{16}$-$C_{30}$-Fettsäuresalzen, insbesondere diejenigen, die sich von Aminen ableiten, wie Triethanolaminstearat; und gegebenenfalls mindestens einem nicht-ionischen Tensid, ausgewählt aus Oxyethylenethern und/oder Oxyp-ropylenethern (die 1 bis 150 Oxyethylen- und/oder Oxypropylengruppen umfassen können) von Fettalkoholen (insbesondere $C_8$-$C_{24}$-Alkohol, und vorzugsweise $C_{12}$-$C_{18}$-Alkohol), wie Stearylalkoholoxyethylenether mit 2 Oxyethylengruppen; Estern von Fettsäure (insbesondere $C_8$-$C_{24}$-Säuren, und vorzugsweise $C_{16}$-$C_{22}$-Säuren) und Polyol, insbesondere von Glycerin und Sorbit, wie Glycerylstearat; Estern von Fettsäure (insbesondere $C_8$-$C_{24}$-Säuren, und vorzugsweise $C_{16}$-$C_{22}$-Säuren) und Oxyethylenglycerinethern und/oder Oxypropylenglycerinethern (die 1 bis 150 Oxyethylengruppen und/oder Oxypropylengruppen umfassen können), wie Polyethoxylglycerylstearat mit 30 Ethylenoxidgruppen und ihren Gemischen.

11. Zusammensetzung nach einem der Ansprüche 9 oder 10, **dadurch gekennzeichnet, dass** sie 0,01 bis 30 Gew.-% Emulgator, insbesondere 1 bis 15 Gew.-% und ganz besonders 2 bis 10 Gew.-% bezogen auf das Gesamtgewicht der Zusammensetzung umfasst.

12. Beschichtungsverfahren für Keratinfasern, umfassend mindestens die folgenden Schritte:

- In-Kontakt-Bringen der Keratinfasern mit einem Teil von mindestens der Oberfläche eines Stifts, umfassend eine kosmetische Zusammensetzung wie nach einem der Ansprüche 1 bis 11 definiert; dann
- Vornehmen einer relativen Verschiebung zwischen der Oberfläche des Stiftes und den Keratinfasern derart, dass der Zerfall des Stifts und die Bildung einer Ablagerung von mindestens einer Schicht der kosmetischen Zusammensetzung auf den Keratinfasern hervorgerufen wird.

**13.** Verpackungs- und Aufbringeinrichtung einer kosmetischen Pflege- und/oder Schminkzusammensetzung für Keratinfasern, **dadurch gekennzeichnet, dass** sie mindestens Folgendes umfasst:

• einen Stift, umfassend eine kosmetische Zusammensetzung nach einem der Ansprüche 1 bis 11;
• einen Träger des Stifts; und
• gegebenenfalls mindestens ein Aufbringelement, wobei das Aufbringelement weiterhin mit Trenn- und/oder Kämmeinrichtungen der Keratinfasern ausgestattet sein kann.

**14.** Trocken auftragbare Zusammensetzung nach Anspruch 1 in Stiftform, die zum Schminken und/oder Pflegen von Keratinfasern ausgelegt ist,

(i) die in Form einer Emulsion vorliegt, umfassend eine in einer wässrigen Phase dispergierte Fettphase, wobei die Fettphase Carnaubawachs und/oder synthetisches Bienenwachs in einem Gehalt von mindestens 2 Gew.-%, vorzugsweise von mindestens 3 Gew.-%, vorteilhafterweise von mindestens 4 Gew.-%, sogar 5 Gew.-% bis 30 Gew.-% bezogen auf das Gesamtgewicht der Zusammensetzung umfasst, wobei die Zusammensetzung einen Trockenextraktgehalt von kleiner oder gleich 45 Gew.-% und größer oder gleich 25 Gew.-% bezogen auf das Gesamtgewicht der Zusammensetzung aufweist, wobei die Zusammensetzung weiterhin mindestens einen Emulgator umfasst, ausgewählt aus anionischen Tensiden, ausgewählt aus Phosphorsäureestern und ihren Salzen, wie Kaliumcetylphosphat,
(ii) die eine Scherhärte zwischen 375 g/m und 5000 g/m aufweist, wobei die Scherhärte bei 20 °C an einem Stift mit Hilfe eines starren Wolframdrahts mit 250 μm Durchmesser gemessen wird, indem der Draht relativ zu dem Stift mit einer Geschwindigkeit von 100 mm/min vorgetrieben wird,
(iii) wobei der Stift durch ein Verfahren erhalten wird, umfassend mindestens die folgenden aufeinanderfolgenden Schritte:

• Erwärmen der Emulsion auf eine Temperatur zwischen 80 °C und 100 °C, insbesondere zwischen 85 °C und 100 °C, beispielsweise eine Temperatur von 95 °C,
• Emulgieren der Phasen mit Hilfe insbesondere eines Rührers, um eine Zusammensetzung vom Typ Emulsion mit kontinuierlicher wässriger Phase zu erhalten,
• Heißfließenlassen der Zusammensetzung in eine Verpackungseinrichtung, und dann
• Vornehmen eines Kälteeintrags, der ein Absenken der Temperatur des Stifts unter 4 °C innerhalb von 30 Minuten ermöglicht.

## Claims

**1.** A cosmetic composition in stick form that can be applied dry for the care and/or make-up of keratin fibres, (i) in the form of an emulsion comprising a fatty phase dispersed in an aqueous phase, said fatty phase containing carnauba wax and/or synthetic beeswax in an amount of at least 2 % by weight relative to the total weight of said composition and (ii) having a shear hardness of between 375 g/m and 5000 g/m, said shear hardness being measured at 20°C on a cylindrical stick using a rigid tungsten wire of diameter 250 μm moved against the stick at a rate of 100 mm/min, said composition having a dry extract content of 45 % or less by weight and 25 % or higher by weight relative to the total weight of the composition,
said composition further comprising at least one emulsifying agent selected from among anionic surfactants selected from among phosphoric esters and the salts thereof such as potassium cetyl phosphate,
said composition able to be obtained or being obtained with a method comprising at least one step comprising heat removal to allow lowering of the temperature of the composition to below 4°C in less than 30 minutes.

**2.** The composition according to the preceding claim 1, **characterized in that** the carnauba wax and/or synthetic beeswax is contained in an amount of at least 3 % by weight, advantageously at least 4 % by weight, even 5 to 30 % by weight relative to the total weight of said composition.

3. The composition according to any of the preceding claims, **characterized in that** the shear hardness is between 500 g/m and 2500 g/m, in particular between 750 g/m and 1500 g/m.

4. The composition according to any of the preceding claims, **characterized in that** it has a dry extract content of 30 % or higher by weight and more particularly 35 % or higher by weight relative to the total weight of the composition.

5. The composition according to any of the preceding claims, **characterized in that** it further comprises at least one additional wax other than the carnauba and synthetic beeswax, selected from among waxes solid at ambient temperature, optionally in the form of an aqueous wax dispersion, of animal, plant, mineral or synthetic origin and mixtures thereof, in particular having a melting point of 45 °C or higher, in particular 55 °C or higher and/or having a tack of 0.7 N.s or higher and a hardness of 3.5 MPa or lower.

6. The composition according to the preceding claim, **characterized in that** the additional wax is selected from among (i) hydrocarbon waxes such as natural beeswax (or bleached beeswax), lanoline wax, Chinese insect waxes, rice wax, candelilla wax, ouricury wax, esparto grass wax, cork fibre wax, sugarcane wax, Japan wax, sumac wax, montan wax, microcrystalline waxes, paraffins, ozokerite, polyethylene waxes, waxes obtained by Fischer-Tropsch synthesis, waxy copolymers and esters thereof, (ii) the waxes obtained by catalytic hydrogenation of animal or plant oils containing linear or branched $C_8$-$C_{32}$ fatty chains such as hydrogenated jojoba oil, hydrogenated sunflower seed oil, hydrogenated castor oil, hydrogenated copra oil and hydrogenated lanoline oil, di-(trimethylol-1,1,1 propane) tetrastearate, di-(trimethylol-1,1,1 propane) tetrabehenate, (iii) silicone waxes such as alkyl or alkoxy-dimethicones having 16 to 45 carbon atoms, (iv) fluorinated waxes, (v) the wax obtained by hydrogenation of olive oil esterified with stearyl alcohol, and (vi) the waxes obtained by hydrogenation of castor oil esterified with cetyl alcohol.

7. The composition according to any of the preceding claims, **characterized in that** it further comprises ozokerite wax.

8. The composition according to any of the preceding claims, **characterized in that** it has a total content of wax(es) of between 2 and 35 % by weight, preferably between 5 and 30 % by weight relative to the total weight of said composition.

9. The composition according to any of the preceding claims, **characterized in that** it further comprises at least one emulsifying agent selected from among anionic, non-ionic, cationic, amphoteric surfactants, polymeric surfactants and the mixtures thereof,
the non-ionic surfactants being selected from among:

- non-ionic surfactants having a HLB of 8 or higher at 25 °C and selected in particular from among ose esters and ethers, oxyethylenated and/or oxypropylenated glycerol ethers, oxyethylenated and/or oxypropylenated fatty alcohol ethers, esters of fatty acids and polyethylene glycol, esters of fatty acids and oxyethylenated and/or oxypropylenated glycerol ethers, esters of fatty acids and oxyethylenated and/or oxypropylenated sorbitol ethers, dimethicone copolyol, dimethicone copolyol benzoate, copolymers of propylene oxide and ethylene oxide, and the mixtures thereof; and
- non-ionic surfactants having a HLB lower than 8 at 25 °C selected in particular from among ose esters and ethers, oxyethylenated and/or oxypropylenated fatty alcohol ethers, the esters of fatty acids and polyol, lecithins, the mixture of cyclomethicone/dimethicone copolyol;

the anionic surfactants being selected from among salts of $C_{16}$-$C_{30}$ fatty acids, in particular triethanolamine stearate, the salts of polyoxyethylenated fatty acids, phosphoric esters and salts thereof, sulfosuccinates, alkyl ether sulfates, isethionates, acylglutamates, soybean derivatives, citrates, proline derivatives, lactylates, sarcosinates, sulfonates and glycinates; and the amphoteric surfactants being selected from among N-acyl-amino acids and amine oxides, silicone surfactants such as dimethicone copolyol phosphates.

10. The composition according to claim 9, **characterized in that** it comprises at least one additional anionic surfactant selected from among the salts of $C_{16}$-$C_{30}$ fatty acids, in particular those derived from amines such as triethanolamine stearate; and optionally at least one non-ionic surfactant selected from among oxyethylenated and/or oxypropylenated ethers (possibly comprising 1 to 150 oxyethylenated and/or oxypropylenated groups) of fatty alcohols (in particular $C_8$-$C_{24}$ alcohols and preferably $C_{12}$-$C_{18}$) such as the oxyethylenated ether of stearyl alcohol with 2 oxyethylenated groups; the esters of fatty acids (in particular $C_8$-$C_{24}$ acids and preferably $C_{16}$-$C_{22}$) and of polyol, in particular glycerol or sorbitol such as glyceryl stearate; the esters of fatty acids (in particular $C_8$-$C_{24}$ acids and preferably $C_{16}$-$C_{22}$) and of oxyethylenated and/or oxypropylenated glycerol ethers (possibly comprising 1 to 150

oxyethylenated and/or oxypropylenated groups) such as polyethoxylated glyceryl stearate with 30 ethylene oxide groups, and the mixtures thereof.

**11.** The composition according to any of claims 9 or 10, **characterized in that** it comprises 0.01 to 30 % by weight of emulsifying agent, in particular 1 to 15 % by weight and more particularly 2 to 10 % by weight relative to the total weight of said composition.

**12.** A method to coat keratin fibres, comprising at least the steps of:

- placing said keratin fibres in contact with at least part of the surface of a stick comprising a cosmetic composition such as defined in any of claims 1 to 11; then
- effecting relative movement between the surface of said stick and said keratin fibres to cause erosion of said stick and the forming of a deposit of at least one layer of said cosmetic composition on said keratin fibres.

**13.** A device for the packaging and application of a keratin fibre care and/or make-up cosmetic composition, **characterized in that** it comprises at least:

• a stick comprising a cosmetic composition such as defined in any of claims 1 to 11;
• a holder for said stick; and
• optionally at least one application element said application element possibly also being provided with members for separating and/or combing said keratin fibres.

**14.** The composition according to claim 1, in the form of a stick for dry application intended for the make-up and/or care of keratin fibres:

(i) in the form of an emulsion comprising a fatty phase dispersed in an aqueous phase, said fatty phase containing carnauba wax and/or synthetic beeswax in an amount of at least 2 %, preferably at least 3 % by weight, advantageously at least 4 % by weight even 5 % to 30 % by weight relative to the total weight of said composition, said composition having a dry extract content of 45 % or lower by weight and 25 % or higher by weight relative to the total weight of the composition, said composition further comprising at least one emulsifying agent selected from among anionic surfactants selected from among phosphoric esters and the salts thereof such as potassium cetyl phosphate;
(ii) having a shear hardness of between 375 g/m and 5000 g/m, said shear hardness being measured at 20°C on a stick using a rigid tungsten wire of diameter 250 μm that is moved against the stick at a rate of 100 mm/minute;
(iii) said stick being obtained with a method comprising at least the following successive steps of:

• heating said emulsion to a temperature of between 80 °C and 100 °C, more particularly between 85°C and 100°C e.g. at a temperature of 95°C;
• emulsifying said phases by means of an agitator in particular to obtain a composition of emulsion type with continuous aqueous phase;
• hot casting said composition into a packaging device; then
• removing heat to lower the temperature of the stick to below 4°C in less than 30 minutes.

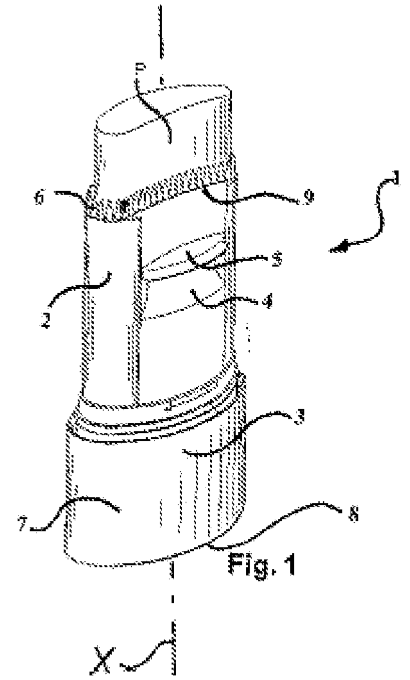


**FIGURE 1**

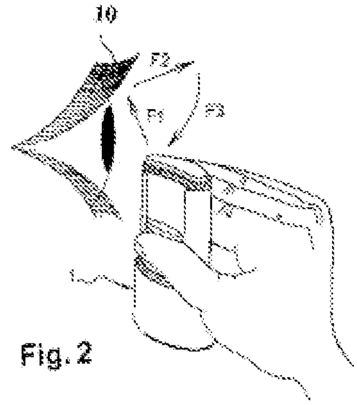


**FIGURE 2**

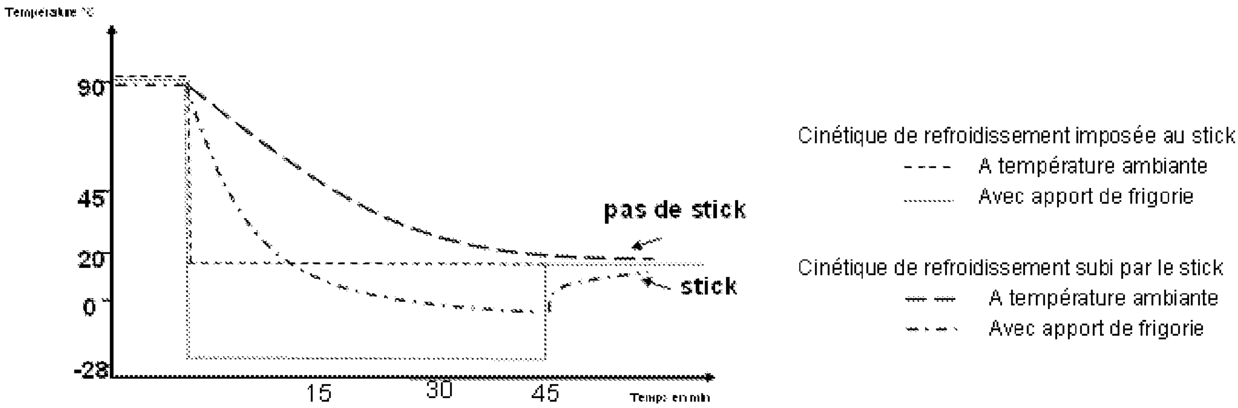
90
45
20
0
-28
15
30
45
pas de stick
stick
Cinétique de refroidissement imposée au stick
A température ambiante
Avec apport de frigorie
Cinétique de refroidissement subi par le stick
A température ambiante
Avec apport de frigorie

FIGURE 3

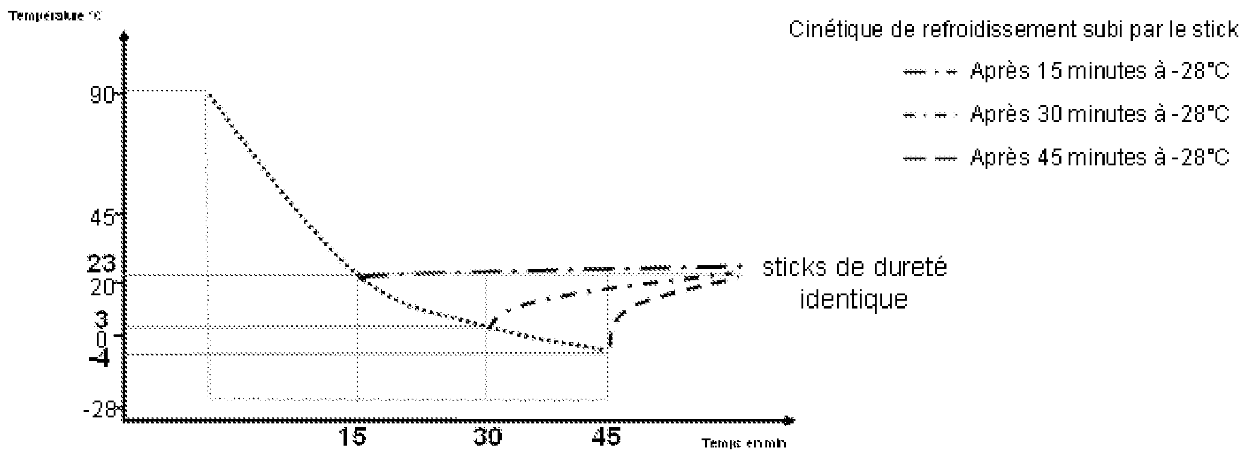
Température °C
Cinétique de refroidissement subi par le stick
Après 15 minutes à -28°C
Après 30 minutes à -28°C
Après 45 minutes à -28°C
90
45
23
20
3
0
-4
-28
sticks de dureté identique
15
30
45
Temps en min

FIGURE 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2007245 A
- FR 2833163
- WO 2006057439 A
- FR 2232303
- EP 1473017 A
- EP 1745771 A
- FR 2792190 A
- EP 1396259 A
- EP 1068854 A
- EP 1086945 A
- WO 0247031 A
- WO 9323008 A
- US 5874069 A
- US 5919441 A
- US 6051216 A
- US 5981680 A
- EP 0955039 A
- FR 2232303 A
- US 5162410 A
- WO 2004073626 A
- EP 1411069 A
- WO 04028488 A
- WO 04055081 A
- WO 0239961 A
- EP 1913835 A


### Littérature non-brevet citée dans la description


- **GRIFFIN.** *J. Soc. Cosm. Chem.,* 1954, vol. 5, 249-256
- **KIRK-OTHMER.** Encyclopedia of Chemical Technology. WILEY, 1979, vol. 22, 333-432
- Microemulsions Theory and Practice. Academic Press, 1977, 21-32
- CTFA. 1995
- **P. TERECH.** Specialist Surfactants. 1997, 209-263